(19) **Europäisches Patentamt European Patent Office Office européen des brevets**

(11) **EP 2 462 446 B1**

(12) **EUROPEAN PATENT SPECIFICATION**

(45) Date of publication and mention of the grant of the patent:
**27.09.2017 Bulletin 2017/39**

(51) Int Cl.:
***G01N 33/566*** (2006.01)   ***G01N 33/68*** (2006.01)

(21) Application number: **10807232.3**

(22) Date of filing: **06.08.2010**

(86) International application number:
**PCT/US2010/044708**

(87) International publication number:
**WO 2011/017614 (10.02.2011 Gazette 2011/06)**

(54) **METHODS AND COMPOSITIONS FOR DIAGNOSIS AND PROGNOSIS OF RENAL INJURY AND RENAL FAILURE**

VERFAHREN UND ZUSAMMENSETZUNGEN ZUR DIAGNOSE UND PROGNOSE VON NIERENVERLETZUNGEN UND NIERENINSUFFIZIENZ

MÉTHODES ET COMPOSITIONS UTILISABLES À DES FINS DE DIAGNOSTIC ET DE PRONOSTIC EN CAS DE LÉSION OU D'INSUFFISANCE RÉNALE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **07.08.2009 US 232318 P**

(43) Date of publication of application:
**13.06.2012 Bulletin 2012/24**

(73) Proprietor: **Astute Medical, Inc.**
**San Diego, CA 92121 (US)**

(72) Inventors:
 • **NEZIERI, Dashurie**
  **A1090 Vienna (AT)**
 • **WAGNER, Ludwig**
  **A1090 Vienna (AT)**
 • **NAKAMURA, Kevin**
  **Cardiff By The Sea,CA 92007 (US)**

(74) Representative: **Schiweck, Weinzierl & Koch Patentanwälte Partnerschaft mbB**
**Landsberger Straße 98**
**80339 München (DE)**

(56) References cited:
 **US-A1- 2008 014 644   US-B1- 6 664 385**

 • **NEZIRI D ET AL: "Cloning and molecular characterization of Dashurin encoded by C20orf116, a PCI-domain containing protein", BIOCHIMICA ET BIOPHYSICA ACTA - GENERAL SUBJECTS, ELSEVIER SCIENCE PUBLISHERS, NL, vol. 1800, no. 4, 1 April 2010 (2010-04-01), pages 430-438, XP026932984, ISSN: 0304-4165, DOI: 10.1016/J.BBAGEN.2009.12.004 [retrieved on 2010-03-02]**
 • **HOSTE ET AL.: 'RIFLE criteria for acute kidney injury are associated with hospital mortality in critically ill patients: a cohort analysis' CRITICAL CARE vol. 10, no. ISSUE, May 2006, XP021020906**

**Description**

The present invention claims priority from U.S. Provisional Patent Application 61/232,318 filed August 7, 2009.

BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830. Renal disease and/or injury may be acute or chronic. Acute and chronic kidney disease are described as follows (from Current Medical Diagnosis & Treatment 2008, 47th Ed, McGraw Hill, New York, pages 785-815): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week) reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222:

| Type | Risk Factors |
| --- | --- |
| **Prerenal** | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |

(continued)

| Intrinsic Renal | |
|---|---|
| Acute tubulo interstitial nephritis | Drug reaction (eg, β-lactams, NSAIDs, sulfonamides, ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |
| Postrenal | |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370, 2005. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004) investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004, proposes the following classifications for stratifying AKI patients:

"Risk": serum creatinine increased 1.5 fold from baseline OR urine production of <0.5 ml/kg body weight for 6 hours;

"Injury": serum creatinine increased 2.0 fold from baseline OR urine production <0.5 ml/kg for 12 h;

"Failure": serum creatinine increased 3.0 fold from baseline OR creatinine >355 $\mu$mol/l (with a rise of >44) or urine output below 0.3 ml/kg for 24 h;

And included two clinical outcomes:

"Loss": persistent need for renal replacement therapy for more than four weeks.

"ESRD": end stage renal disease-the need for dialysis for more than 3 months.

These criteria are called the RIFFLE criteria.

More recently, Mehta et al., Crit. Care 11:R31 (doi:10.1186.cc5713), 2007, proposes the following similar classifications for stratifying AKI patients:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR urine output less than 0.3 mL/kg per hour for 24 hours or anuria for 12 hours.

The CIN Consensus Working Panel (McCollough et al, Rev Cardiovasc Med. 2006; 7(4):177-197) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

US 6 664 385 B1 describes inter alia proteins that are upregulated in injured and regenerating tissues, as well as DNA encoding these proteins. In particular, Kidney Injury-related molecules ("KIM") are shown to be upregulated in renal tissue after injury to the kidney. US 2008/014644 A1 describes a method of assessing the ongoing kidney status of a mammal afflicted with or at risk of developing chronic renal injury or disease, including chronic renal failure (CRF) by detecting the quantity of Neutrophil Gelatinase-Associated Lipocalin (NGAL) in urine, serum or plasma samples at discrete time periods, as well as over time. Incremental increases in NGAL levels in CRF patients over a prolonged period of time are described to be diagnostic of worsening kidney disease. Hoste et al. (2006) "RIFLE criteria for acute kidney injury are associated with hospital mortality in critically ill patients: a cohort analysis" Critical Care Vol. 10: R73 describes inter alia that RIFLE criteria for acute kidney injury are associated with hospital mortality in critically ill patients.

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Knowing these things can be of vital importance in managing and treating patients with AKI.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

BRIEF SUMMARY OF THE INVENTION

It is an object of the disclosure to provide methods and compositions for evaluating renal function in a subject. As described herein, measurement of Dashurin can be used for diagnosis, prognosis, staging, monitoring, categorizing and determination of further diagnosis and treatment regimens in subjects suffering or at risk of suffering from an injury to renal function, reduced renal function, and/or acute renal failure (also called acute kidney injury).

This biomarker may be used for risk stratification (that is, to identify subjects at risk for a future injury to renal function, for future progression to reduced renal function, for future progression to ARF, for future improvement in renal function, *etc.*); for diagnosis of existing disease (that is, to identify subjects who have suffered an injury to renal function, who have progressed to reduced renal function, who have progressed to ARF, *etc.*); for monitoring for deterioration or improvement of renal function; and for predicting a future medical outcome, such as improved or worsening renal function, a decreased or increased mortality risk, a decreased or increased risk that a subject will require dialysis, a decreased or increased risk that a subject will require renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will suffer rejection of a transplanted kidney, *etc.*

In a first aspect, the present invention relates to a method for evaluating renal status in a subject, comprising:

performing an assay method configured to detect one or more biomarkers comprising Dashurin on a body fluid sample obtained from the subject to provide an assay result,
wherein the assay generates a detectable signal indicative of the presence or amount of a physiologically relevant concentration of Dashurin of SEQ ID NO: 1 and/or immunoreactive fragments thereof; and
correlating the assay result to the renal status of the subject.

In a second aspect, the present invention relates to the use of Dashurin of SEQ ID NO:1 and/or immunoreactive fragments thereof for the diagnosis, risk stratification, prognosis, classifying and monitoring of renal status of a subject not receiving renal replacement therapy,
for the diagnosis, risk stratification, prognosis, classifying and monitoring of renal status of a subject not in acute renal failure, or
for assigning an increased likelihood of progressing to a worsening RIFLE stage to a subject, relative to the subject's current RIFLE stage.

In a third aspect the present invention relates to a kit, comprising: reagents for performing an assay method configured to detect one or more biomarkers comprising Dashurin, wherein the assay method generates a detectable signal indicative of the presence or amount of a physiologically relevant concentration of Dashurin of SEQ ID NO: 1 and/or immunoreactive fragments thereof.

Also disclosed are methods for evaluating renal status in a subject. These methods comprise performing an assay method that is configured to detect Dashurin in a body fluid sample obtained from the subject. The assay result, for example a measured concentration of Dashurin, is then correlated to the renal status of the subject. This correlation to renal status may include correlating the assay result to one or more of risk stratification, diagnosis, staging, classifying and monitoring of the subject as described herein. Thus, the methods utilize Dashurin for the evaluation of renal injury.

In certain embodiments, the methods for evaluating renal status described herein are methods for risk stratification of the subject; that is, assigning a likelihood of one or more future changes in renal status to the subject. In these embodiments, the assay result is correlated to one or more such future changes. The following are preferred risk stratification embodiments.

In preferred risk stratification embodiments, these methods comprise determining a subject's risk for a future injury to renal function, and the assay result, for example a measured concentration of Dashurin, is correlated to a likelihood of such a future injury to renal function. For example, the measured concentration may be compared to a threshold value, and an increased likelihood of suffering a future injury to renal function is assigned to the subject when the measured concentration is above the threshold, relative to a likelihood assigned when the measured concentration is below the threshold.

In other preferred risk stratification embodiments, these methods comprise determining a subject's risk for future reduced renal function, and the assay result, for example a measured concentration of Dashurin, is correlated to a likelihood of such reduced renal function. For example, the measured concentration may be compared to a threshold value, and an increased likelihood of future reduced renal function is assigned to the subject when the measured concentration is above the threshold, relative to a likelihood assigned when the measured concentration is below the threshold.

In still other preferred risk stratification embodiments, these methods comprise determining a subject's likelihood for a future improvement in renal function, and the assay result, for example a measured concentration of Dashurin, is correlated to a likelihood of such a future improvement in renal function. For example, the measured concentration may be compared to a threshold value, and an increased likelihood of a future improvement in renal function is assigned to the subject when the measured concentration is below the threshold, relative to a likelihood assigned when the measured concentration is above the threshold.

In yet other preferred risk stratification embodiments, these methods comprise determining a subject's risk for progression to ARF, and the assay result, for example a measured concentration of Dashurin, is correlated to a likelihood of such progression to ARF. For example, the measured concentration may be compared to a threshold value, and an increased likelihood of progression to ARF is assigned to the subject when the measured concentration is above the threshold, relative to a likelihood assigned when the measured concentration is below the threshold.

And in other preferred risk stratification embodiments, these methods comprise determining a subject's outcome risk, and the assay result, for example a measured concentration of Dashurin, is correlated to a likelihood of the occurrence of a clinical outcome related to a renal injury suffered by the subject. For example, the measured concentration may be compared to a threshold value, and an increased likelihood of mortality, a requirement for dialysis, a requirement for renal transplantation, a requirement for withdrawal of renal toxins, an increased likelihood of end stage renal disease, an increased likelihood of heart failure, an increased likelihood of stroke, an increased likelihood of mortality, an increased likelihood of myocardial infarction, *etc.,* is assigned to the subject when the measured concentration is above the threshold, relative to a likelihood assigned when the measured concentration is below the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 120 days, 90 days, 60 days, 45 days, 30 days, 21 days, 14 days, 7 days, 5 days, 96 hours, 72 hours, 48 hours, 36 hours, 24 hours, 12 hours, or less.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of Dashurin measured in such normal subjects. Alternatively, the threshold value may be determined from a population of subjects suffering from an injury to renal function or reduced renal function, but which do not progress to ARF (or some other clinical outcome such as death, dialysis, renal transplantation, *etc.*) by selecting a concentration representing the 75th, 85th, 90th, 95th, or 99th percentile of Dashurin measured in such subjects. In another alternative, the threshold value may be determined from a prior measurement of Dashurin in the same subject; that is, a temporal change in the level of Dashurin in the subject may be used to assign risk to the subject. That is, an increasing concentration of Dashurin over time may be used to identify subjects at increased risk for future injury or progression of an existing renal injury; relative to a risk assigned when the concentration is stable or decreasing.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of Dashurin is treated as a continuous variable. For example, any particular concentration can be converted into a probability of a future reduction in renal function for the subject. In yet another alternative, a threshold that can provide an acceptable level of specificity and sensitivity in separating a population of subjects into a "first" subpopulation which is predisposed to one or more future changes in renal status, and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95;

a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95;

at least 75% sensitivity, combined with at least 75% specificity;

a positive likelihood ratio (calculated as sensitivity/(1-specificity)) of greater than 1, at least 2, more preferably at least 3, still more preferably at least 5, and most preferably at least 10; or

a negative likelihood ratio (calculated as (1-sensitivity)/specificity) of less than 1, less than or equal to 0.5, more preferably less than or equal to 0.3, and most preferably less than or equal to 0.1.

The term "about" in the context of any of the above measurements refers to +/- 5% of a given measurement.

Multiple thresholds may also be used to assess renal status in a subject. For example, a "first" subpopulation which is predisposed to one or more future changes in renal status, and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind the full length Dashurin protein, and may also bind one or more polypeptides that are "related" thereto, as that term is defined hereinafter. Numerous immunoassay formats are known to those of skill in the art. Preferred body fluid samples are selected from the group consisting of urine, blood, serum, and plasma.

The foregoing method steps should not be interpreted to mean that the Dashurin assay result is used in isolation for risk stratification of a subject. Rather, additional variables or other clinical indicia may be included in the methods described herein. For example, a risk stratification method may combine the assay result with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma BNP concentration, a serum or plasma NTproBNP concentration, and a serum or plasma proBNP concentration. Other measures of renal function which may be combined with the Dashurin assay result are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

Such additional variables may be measured in samples obtained at the same time as the sample used to determine the Dashurin assay result, or may be determined at an earlier or later time. The additional variables may also be measured on the same or different body fluid samples. For example, Dashurin may be measured in a serum or plasma sample and another biomarker may be measured in a urine sample. In addition, assignment of a likelihood may combine the Dashurin assay result with temporal changes in one or more such additional variables.

In other embodiments, the methods for evaluating renal status described herein are methods for diagnosing a renal injury in the subject; that is, assessing whether or not a subject has suffered from an injury to renal function, reduced renal function, or ARF. In these embodiments, the assay result is correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the assay result, for example a measured concentration of Dashurin, is correlated to the occurrence or nonoccurrence of such an injury. For example, the measured concentration may be compared to a threshold value, and when the measured concentration is above the threshold, an increased likelihood of the occurrence of an injury to renal function is assigned to the subject relative to the likelihood assigned when the measured concentration is below the threshold; alternatively, when the measured concentration is below the threshold, a decreased likelihood of the nonoccurrence of an injury to renal function is assigned to the subject relative to the likelihood assigned when the measured concentration is above the threshold. As noted hereinafter, a "diagnosis" using the Dashurin assay result can take into consideration of other clinical indicia.

In other preferred diagnostic embodiments, these methods comprise diagnosing the occurrence or nonoccurrence of reduced renal function, and the assay result, for example a measured concentration of Dashurin, is correlated to the occurrence or nonoccurrence of an injury causing reduced renal function. For example, the measured concentration may be compared to a threshold value, and when the measured concentration is above the threshold, an increased likelihood of the occurrence of an injury causing reduced renal function is assigned to the subject relative to the likelihood assigned when the measured concentration is below the threshold; alternatively, when the measured concentration is below the threshold, a decreased likelihood of the nonoccurrence of an injury causing reduced renal function is assigned to the subject relative to the likelihood assigned when the measured concentration is above the threshold.

In yet other preferred diagnostic embodiments, these methods comprise diagnosing the occurrence or nonoccurrence of ARF, and the assay result, for example a measured concentration of Dashurin, is correlated to the occurrence or nonoccurrence of an injury causing ARF. For example, the measured concentration may be compared to a threshold value, and when the measured concentration is above the threshold, an increased likelihood of the occurrence of an injury causing ARF is assigned to the subject relative to the likelihood assigned when the measured concentration is below the threshold; alternatively, when the measured concentration is below the threshold, a decreased likelihood of the nonoccurrence of an injury causing ARF is assigned to the subject relative to the likelihood assigned when the measured concentration is above the threshold.

In still other preferred diagnostic embodiments, these methods comprise diagnosing a subject as being in need of dialysis, and the assay result, for example a measured concentration of Dashurin, is correlated to a need for dialysis. For example, the measured concentration may be compared to a threshold value, and when the measured concentration is above the threshold, an increased likelihood of a present need for dialysis is assigned to the subject relative to the likelihood assigned when the measured concentration is below the threshold; alternatively, when the measured concentration is below the threshold, a decreased likelihood of a present need for dialysis is assigned to the subject relative to the likelihood assigned when the measured concentration is above the threshold.

In still other preferred diagnostic embodiments, these methods comprise diagnosing a subject as being in need of renal transplantation, and the assay result, for example a measured concentration of Dashurin, is correlated to a need for renal transplantation. For example, the measured concentration may be compared to a threshold value, and when the measured concentration is above the threshold, an increased likelihood of a present need for renal transplantation is assigned to the subject relative to the likelihood assigned when the measured concentration is below the threshold; alternatively, when the measured concentration is below the threshold, a decreased likelihood of a present need for renal transplantation is assigned to the subject relative to the likelihood assigned when the measured concentration is above the threshold.

A variety of methods may be used by the skilled artisan to arrive at a desired threshold value. For example, the threshold value may be determined from a population of normal subjects by selecting a concentration representing the 75th, 85th, 90th, 95th, or 99th percentile of Dashurin measured in such normal subjects. In another alternative, the threshold value may be determined from a prior measurement of Dashurin in the same subject; that is, a temporal change in the level of Dashurin in the subject may be used to assign a diagnosis to the subject.

In yet another alternative, a threshold that can provide an acceptable level of specificity and sensitivity in separating a combined population of subjects into a "first" subpopulation having a disease of interest, and a "second" subpopulation not having a disease of interest. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95;

a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95;

at least 75% sensitivity, combined with at least 75% specificity;

a positive likelihood ratio (calculated as sensitivity/(1-specificity)) of greater than 1, at least 2, more preferably at least 3, still more preferably at least 5, and most preferably at least 10; or

a negative likelihood ratio (calculated as (1-sensitivity)/specificity) of less than 1, less than or equal to 0.5, more preferably less than or equal to 0.3, and most preferably less than or equal to 0.1.

The term "about" in this context refers to +/- 5% of a given measurement.

Multiple thresholds may also be used to assess renal status in a subject. For example, a "first" subpopulation of individuals having a condition of interest, and a "second" subpopulation which does not can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to have the condition in comparison to someone in the first tertile. The third tertile is also assigned an odds ratio that is relative to that first tertile.

In certain embodiments, the assay method is an immunoassay. Antibodies for use in such assays will specifically bind the full length Dashurin protein, and may also bind one or more polypeptides that are "related" thereto, as that term is defined hereinafter. Numerous immunoassay formats are known to those of skill in the art. Preferred body fluid samples are selected from the group consisting of urine, blood, serum, and plasma.

The foregoing method steps should not be interpreted to mean that the Dashurin assay result is used in isolation for diagnosis of a subject. Rather, additional variables may be included in the methods described herein. For example, a diagnosis may combine the assay result with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma BNP concentration, a serum or plasma NTproBNP concentration, and a serum or plasma proBNP concentration. Other measures of renal function which may be combined with the Dashurin assay result are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

Such additional variables may be measured in samples obtained at the same time as the sample used to determine the Dashurin assay result, or may be determined at an earlier or later time. The additional variables may also be measured on the same or different body fluid samples. For example, Dashurin may be measured in a serum or plasma sample and another biomarker may be measured in a urine sample. In addition, a diagnostic method may combine the Dashurin assay result with temporal changes in one or more such additional variables.

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay result is correlated to the occurrence or nonoccurrence of a change in renal status. The following are preferred monitoring embodiments.

In preferred monitoring embodiments, these methods comprise monitoring renal status in a subject suffering from an injury to renal function, and the assay result, for example a measured concentration of Dashurin, is correlated to the occurrence or nonoccurrence of a change in renal status in the subject. For example, the measured concentration may be compared to a threshold value, and when the measured concentration is above the threshold, a worsening of renal function may be assigned to the subject; alternatively, when the measured concentration is below the threshold, an improvement of renal function may be assigned to the subject.

In other preferred monitoring embodiments, these methods comprise monitoring renal status in a subject suffering from reduced renal function, and the assay result, for example a measured concentration of Dashurin, is correlated to the occurrence or nonoccurrence of a change in renal status in the subject. For example, the measured concentration may be compared to a threshold value, and when the measured concentration is above the threshold, a worsening of renal function may be assigned to the subject; alternatively, when the measured concentration is below the threshold, an improvement of renal function may be assigned to the subject.

In yet other preferred monitoring embodiments, these methods comprise monitoring renal status in a subject suffering from acute renal failure, and the assay result, for example a measured concentration of Dashurin, is correlated to the occurrence or nonoccurrence of a change in renal status in the subject. For example, the measured concentration may be compared to a threshold value, and when the measured concentration is above the threshold, a worsening of renal function may be assigned to the subject; alternatively, when the measured concentration is below the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result, for example a measured concentration of Dashurin, is correlated to the occurrence or nonoccurrence of a change in renal status in the subject. For example, the measured concentration may be compared to a threshold value, and when the measured concentration is above the threshold, a worsening of renal function may be assigned to the subject; alternatively, when the measured concentration is below the threshold, an improvement of renal function may be assigned to the subject.

A variety of methods may be used by the skilled artisan to arrive at a desired threshold value. For example, the threshold value may be determined from a population of normal subjects by selecting a concentration representing the 75th, 85th, 90th, 95th, or 99th percentile of Dashurin measured in such normal subjects. In another alternative, the threshold value may be determined from a prior measurement of Dashurin in the same subject; that is, a temporal change in the level of Dashurin in the subject may be used to assign a diagnosis to the subject.

In yet another alternative, a threshold that can provide an acceptable level of specificity and sensitivity in separating a combined population of subjects into a "first" subpopulation having a disease of interest and whose renal function worsens, and a "second" subpopulation having a disease of interest and whose renal function improves. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95;

a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95;

at least 75% sensitivity, combined with at least 75% specificity;

a positive likelihood ratio (calculated as sensitivity/(1-specificity)) of greater than 1, at least 2, more preferably at least 3, still more preferably at least 5, and most preferably at least 10; or

a negative likelihood ratio (calculated as (1-sensitivity)/specificity) of less than 1, less than or equal to 0.5, more preferably less than or equal to 0.3, and most preferably less than or equal to 0.1.

The term "about" in this context refers to +/- 5% of a given measurement.

Multiple thresholds may also be used to monitor renal status in a subject. For example, a "first" subpopulation of individuals in which renal function is improving (or worsening), and a "second" subpopulation of individuals which do not can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to improve (or worsen) in comparison to someone in the first tertile. The third tertile is also assigned an odds ratio that is relative to that first tertile.

In certain embodiments, the assay method is an immunoassay. Antibodies for use in such assays will specifically bind the full length Dashurin protein, and may also bind one or more polypeptides that are "related" thereto, as that term is defined hereinafter. Numerous immunoassay formats are known to those of skill in the art. Preferred body fluid samples are selected from the group consisting of urine, blood, serum, and plasma.

The foregoing method steps should not be interpreted to mean that the Dashurin assay result is used in isolation for monitoring a subject. Rather, additional variables may be included in the methods described herein. For example, a monitoring may combine the assay result with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma BNP concentration, a serum or plasma NTproBNP concentration, and a serum or plasma proBNP concentration. Other measures of renal function which may be combined with the Dashurin assay result are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

Such additional variables may be measured in samples obtained at the same time as the sample used to determine the Dashurin assay result, or may be determined at an earlier or later time. The additional variables may also be measured on the same or different body fluid samples. For example, Dashurin may be measured in a serum or plasma sample and another biomarker may be measured in a urine sample. In addition, a monitoring method may combine the Dashurin assay result with temporal changes in one or more such additional variables.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease. In these embodiments, the assay result is correlated to a particular class and/or subclass. The following are preferred classification embodiments.

In preferred classification embodiments, these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease, and the assay result, for example a measured concentration of Dashurin, is correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value. For example, the threshold value may be determined from a population of subjects not having a particular classification (e.g., a "control" group) by selecting a concentration representing the 75th, 85th, 90th, 95th, or 99th percentile of Dashurin measured in such control subjects. In another alternative, the threshold value may be determined from a prior measurement of Dashurin in the same subject; that is, a temporal change in the level of Dashurin in the subject may be used to assign a classification to the subject.

In yet another alternative, a threshold that can provide an acceptable level of specificity and sensitivity in separating a combined population of subjects into a "first" subpopulation having a particular classification, and a "second" subpopulation having a different classification. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95;

a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95;

at least 75% sensitivity, combined with at least 75% specificity;

a positive likelihood ratio (calculated as sensitivity/(1-specificity)) of greater than 1, at least 2, more preferably at least 3, still more preferably at least 5, and most preferably at least 10; or

a negative likelihood ratio (calculated as (1-sensitivity)/specificity) of less than 1, less than or equal to 0.5, more preferably less than or equal to 0.3, and most preferably less than or equal to 0.1.

The term "about" in this context refers to +/- 5% of a given measurement.

Multiple thresholds may also be used to classify a renal injury in a subject. For example, a "first" subpopulation of individuals having a particular classification, and a "second" subpopulation having a different classification, can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to have a particular classification in comparison to someone in the first tertile. The third tertile is also assigned an odds ratio that is relative to that first tertile.

In certain embodiments, the assay method is an immunoassay. Antibodies for use in such assays will specifically bind the full length Dashurin protein, and may also bind one or more polypeptides that are "related" thereto, as that term is defined hereinafter. Numerous immunoassay formats are known to those of skill in the art. Preferred body fluid samples are selected from the group consisting of urine, blood, serum, and plasma.

The foregoing method steps should not be interpreted to mean that the Dashurin assay result is used in isolation for classification of an injury in a subject. Rather, additional variables may be included in the methods described herein. For example, a classification may combine the assay result with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma BNP concentration, a serum or plasma NTproBNP concentration, and a serum or plasma proBNP concentration. Other measures of renal function which may be combined with the Dashurin assay result are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

Such additional variables may be measured in samples obtained at the same time as the sample used to determine the Dashurin assay result, or may be determined at an earlier or later time. The additional variables may also be measured on the same or different body fluid samples. For example, Dashurin may be measured in a serum or plasma sample and another biomarker may be measured in a urine sample. In addition, a monitoring method may combine the Dashurin assay result with temporal changes in one or more such additional variables.

In various related aspects, also disclosed are devices and kits for performing the methods described herein. Suitable kits comprise reagents sufficient for performing at least one of the described Dashurin assays, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, etc.) as well as molecules that may be indirectly detected by production of a detectable reaction product (e.g., enzymes such as horseradish peroxidase, alkaline phosphatase, etc.) or through the use of a specific binding molecule which itself may be detectable (e.g., a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, etc. In certain of these methods, the solid phase antibody is coupled to a transducer (e.g., a diffraction grating, electrochemical sensor, etc) for generation of a signal, while in others, a signal is generated by a transducer that is spatially separate from the solid phase antibody (e.g., a fluorometer that employs an excitation light source and an optical detector). This list is not meant to be limiting. Antibody-based biosensors may also be employed to determine the presence or amount of analytes that optionally eliminate the need for a labeled molecule.

BRIEF DESCRIPTION OF THE FIGURES

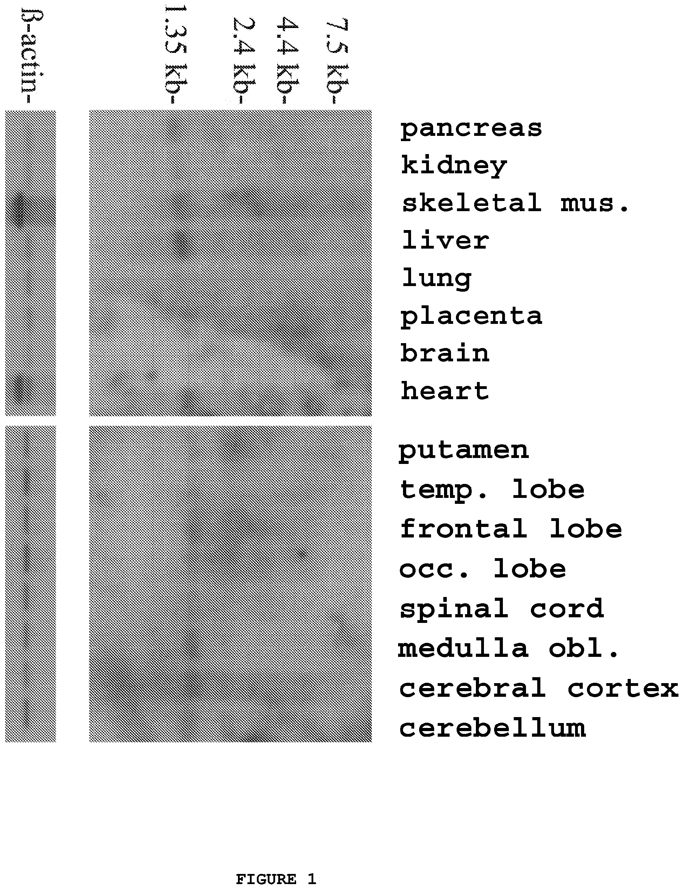

Fig. 1. Multiple tissue Northern analysis of Dashurin. Human mRNA from various human tissues was visualised with 32P-labelled human Dashurin cDNA. The same blot was rehybridized with a β-actin-specific probe for determination of comparable RNA loading. Molecular size markers are indicated at the left. The housekeeping gene β-actin is provided for comparison.

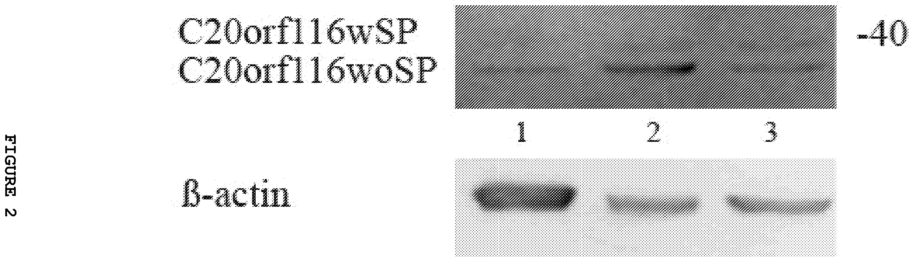

Fig. 2. Immunoblot using mouse anti-Dashurin Ab and *ex vivo* tissue. Tissue was extracted and loaded onto SDS-Page blotted onto nitrocellulose and developed with Dashurin Ab followed by HRP labelled goat-anti-mouse Ab. The housekeeping gene β-actin is provided for comparison.

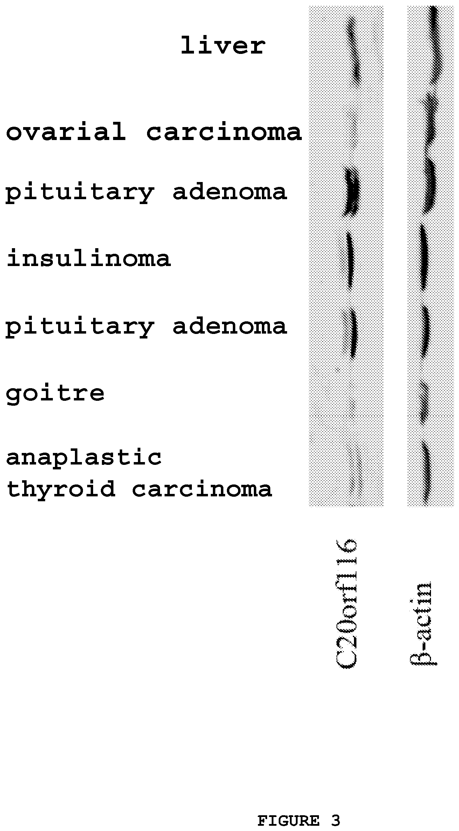

Fig. 3. Immunoblot using mouse anti-Dashurin Ab and *ex vivo* tumor tissue. Tissue and tumor cell extract was loaded after removing the cell debris by centrifugation onto 12% SDS-PAGE and transferred to nitrocellulose. The blotted membrane was developed consecutively by mouse anti- human Dashurin Ab (upper panel) and β-actin (lower panel).

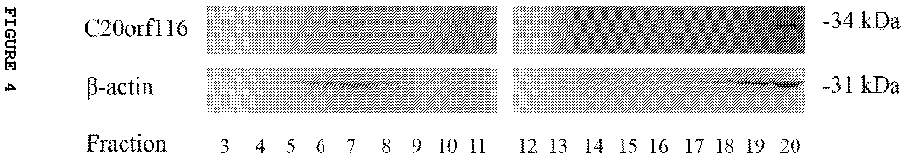

Fig. 4. HepG2 cell fractionation. Hep G2 cells were scraped into PBS containing protease inhibitor and the cell suspension was drawn four times through a 26-gauge needle followed by centrifugation at 2500 RPM for pelleting nuclei. The supernatant was loaded onto a discontinuous sucrose gradient and spun for 3h. The collected fractions from various layers were loaded onto a 12% SDS-PAGE and blotted consecutively for Dashurin and β-actin.

DETAILED DESCRIPTION OF THE INVENTION

The present disclosure relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of Dashurin.

For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Suitable measures include a decrease in glomerular filtration rate or estimated GFR, a reduction in urine output, an increase in serum creatinine, an increase in serum cystatin C, a requirement for dialysis, *etc.* "Improvement in Renal Function" is an abrupt (within 14 days, preferably within 7 days, more preferably within 72 hours, and still more preferably within 48 hours) measurable increase in such a measure of renal function. Preferred measures for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq$ 8.8 μmol/L), a percentage increase in serum creatinine of greater than or equal to 20% (1.2-fold from baseline), or a reduction in urine output (documented oliguria of less than 0.25 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq$ 26.4 μmol/l), a percentage increase in serum creatinine of greater than or equal to 25% (1.25-fold from baseline), or a reduction in urine output (documented oliguria of less than 0.5 ml/kg per hour). This term is synonymous with "acute kidney injury" or "AKI."

The present invention relates in part to isolated Dashurin polypeptides and isolated nucleic acids encoding such polypeptides. As used herein, the term "Dashurin" refers to one or more polypeptides present in a biological sample that are derived from the Dashurin precursor having the following amino acid sequence (SEQ ID NO: 1):

```
1      M  V  A  P  V  W  Y  L  V  A  A  A  L  L  V  G  F  I  L  F
21     L  T  R  S  R  G  R  A  A  S  A  G  Q  E  P  L  H  N  E  E
41     L  A  G  A  G  R  V  A  Q  P  G  P  L  E  P  E  E  P  R  A
61     G  G  R  P  R  R  R  R  D  L  G  S  R  L  Q  A  Q  R  R  A
81     Q  R  V  A  W  A  E  A  D  E  N  E  E  E  A  V  I  L  A  Q
101    E  E  E  G  V  E  K  P  A  E  T  H  L  S  G  K  I  G  A  K
121    K  L  R  K  L  E  E  K  Q  A  R  K  A  Q  R  E  A  E  E  A
141    E  R  E  E  R  K  R  L  E  S  Q  R  E  A  E  W  K  K  E  E
161    E  R  L  R  L  E  E  E  Q  K  E  E  E  E  R  K  A  R  E  E
181    Q  A  Q  R  E  H  E  E  Y  L  K  L  K  E  A  F  V  V  E  E
201    E  G  V  G  E  T  M  T  E  E  Q  S  Q  S  F  L  T  E  F  I
221    N  Y  I  K  Q  S  K  V  V  L  L  E  D  L  A  S  Q  V  G  L
241    R  T  Q  D  T  I  N  R  I  Q  D  L  L  A  E  G  T  I  T  G
261    V  I  D  D  R  G  K  F  I  Y  I  T  P  E  E  L  A  A  V  A
281    N  F  I  R  Q  R  G  R  V  S  I  A  E  L  A  Q  A  S  N  S
301    L  I  A  W  G  R  E  S  P  A  Q  A  P  A
```

which may be encoded by the accompanying nucleic acid sequence (SEQ ID NO: 2) as shown below:

```
1      ATGGTGGCGCCTGTGTGGTACTTGGTAGCGGCGGCTCTGCTAGTCGGCTTTATCCTCTTC
1       M  V  A  P  V  W  Y  L  V  A  A  A  L  L  V  G  F  I  L  F

61     CTGACTCGCAGCCGGGGCCGGGCGGCATCAGCCGGCCAAGAGCCACTGCACAATGAGGAG
21      L  T  R  S  R  G  R  A  A  S  A  G  Q  E  P  L  H  N  E  E
```

```
121 CTGGCAGGAGCAGGCCGGGTGGCCCAGCCTGGGCCCCTGGAGCCTGAGGAGCCGAGAGCT
 41   L  A  G  A  G  R  V  A  Q  P  G  P  L  E  P  E  E  P  R  A

181 GGAGGCAGGCCTCGGCGCCGGAGGGACCTGGGCAGCCGCCTACAGGCCCAGCGTCGAGCC
 61   G  G  R  P  R  R  R  R  D  L  G  S  R  L  Q  A  Q  R  R  A

241 CAGCGGGTGGCCTGGGCAGAAGCAGATGAGAACGAGGAGGAAGCTGTCATCCTAGCCCAG
 81   Q  R  V  A  W  A  E  A  D  E  N  E  E  E  A  V  I  L  A  Q

301 GAGGAGGAAGGTGTCGAGAAGCCAGCGGAAACTCACCTGTCGGGGAAAATTGGAGCTAAG
101   E  E  E  G  V  E  K  P  A  E  T  H  L  S  G  K  I  G  A  K

361 AAACTGCGGAAGCTGGAGGAGAAACAAGCGCGAAAGGCCCAGCGTGAGGCAGAGGAGGCT
121   K  L  R  K  L  E  E  K  Q  A  R  K  A  Q  R  E  A  E  E  A

421 GAACGTGAGGAGCGGAAACGACTCGAGTCCCAGCGCGAAGCTGAGTGGAAGAAGGAGGAG
141   E  R  E  E  R  K  R  L  E  S  Q  R  E  A  E  W  K  K  E  E

481 GAGCGGCTTCGCCTGGAGGAGGAGCAGAAGGAGGAGGAGGAGAGGAAGGCCCGCGAGGAG
161   E  R  L  R  L  E  E  E  Q  K  E  E  E  E  R  K  A  R  E  E

541 CAGGCCCAGCGGGAGCATGAGGAGTACCTGAAACTGAAGGAGGCCTTTGTGGTGGAGGAG
181   Q  A  Q  R  E  H  E  E  Y  L  K  L  K  E  A  F  V  V  E  E

601 GAAGGCGTAGGAGAGACCATGACTGAGGAACAGTCCCAGAGCTTCCTGACAGAGTTCATC
201   E  G  V  G  E  T  M  T  E  E  Q  S  Q  S  F  L  T  E  F  I

661 AACTACATCAAGCAGTCCAAGGTTGTGCTCTTGGAAGACCTGGCTTCCCAGGTGGGCCTA
221   N  Y  I  K  Q  S  K  V  V  L  L  E  D  L  A  S  Q  V  G  L

721 CGCACTCAGGACACCATAAATCGCATCCAGGACCTGCTGGCTGAGGGGACTATAACAGGT
241   R  T  Q  D  T  I  N  R  I  Q  D  L  L  A  E  G  T  I  T  G

781 GTGATTGACGACCGGGGCAAGTTCATCTACATAACCCCAGAGGAACTGGCCGCCGTGGCC
261   V  I  D  D  R  G  K  F  I  Y  I  T  P  E  E  L  A  A  V  A

841 AACTTCATCCGACAGCGGGGCCGGGTGTCCATCGCCGAGCTTGCCCAAGCCAGCAACTTC
281   N  F  I  R  Q  R  G  R  V  S  I  A  E  L  A  Q  A  S  N  S

901 CTCATCGCCTGGGGCCGGGAGTCCCCTGCCCAAGCCCCAGCCTGA
301   L  I  A  W  G  R  E  S  P  A  Q  A  P  A
```

Analysis of the Dashurin gene product demonstrates this as open reading frame of 945 bp coding for 314 amino acids. It is believed that the cleavage site for the signal peptidase is after amino acid residue 28. Thus, the following domains have been identified in Dashurin:

| Residues | Length | Domain ID |
|----------|--------|-----------|
| 1-28 | 28 | Signal peptide |
| 29-314 | 286 | Mature Dashurin |

The skilled artisan will understand that the signals obtained from an immunoassay are a direct result of complexes formed between one or more antibodies and the target biomolecule (*i.e.*, the analyte) and polypeptides containing the necessary epitope(s) to which the antibodies bind. While such assays may detect the full length Dashurin molecule and the assay result be expressed as a concentration of Dashurin, the signal from the assay is actually a result of all such "immunoreactive" polypeptides present in the sample.

Full length Dashurin and immunoreactive polypeptides that are fragments thereof may be used as immunogens to prepare appropriate antibodies to the Dashurin protein, as assay standard material, *etc.* Thus, in addition to Dashurin itself, the present invention relates to isolated polypeptides having a sequence which comprises at least 25 contiguous amino acid residues of SEQ ID NO: 1. In various embodiments, such isolated polypeptides comprise at least 30, at least 50, at least 75, at least 100, at least 150, at least 200, at least 250, or at least all 286 contiguous amino acid residues of SEQ ID NO: 1.

The present invention also includes isolated nucleic acids encoding such polypeptides. Thus, the present invention relates to isolated nucleic acids encoding a polypeptide sequence which comprises at least 25 contiguous amino acid residues of SEQ ID NO: 1. In various embodiments, such isolated polypeptides comprise at least 30, at least 50, at least 75, at least 100, at least 150, at least 200, at least 250, or at least 286 contiguous amino acid residues of SEQ ID NO: 1. These nucleic acids preferably encode at least 75, at least 90, at least 150, at least 225, at least 300, at least 450, at least 600, or at least 858 contiguous nucleic acid residues of SEQ ID NO: 2.

Nucleic acids may be provided as a component of an expression vector, in which the isolated nucleic acid encoding all or a portion of the Dashurin polypeptide is operably linked to appropriate control sequences (e.g., a promoter) for expression of the polypeptide. The term "expression vector" as used herein refers to vectors that have the ability to express heterologous DNA fragments in a foreign cell. Many prokaryotic and eukaryotic expression vectors are known and/or commercially available. Selection of appropriate expression vectors is within the knowledge of those having skill in the art.

The term "isolated" as used herein refers to a molecule that is has been removed from its natural environment. For example, a naturally-occurring polypeptide genomically expressed in a tissue of a living animal is not isolated, but the same polypeptide, separated from some or all of the coexisting materials in the natural system, is isolated. A polypeptide which is present in a system because of recombinant expression is also "isolated" as that term is used herein. For the sake of clarity, the term "isolated" not require absolute purity.

Such polypeptides (and the nucleic acids which encode them) may also include residues from other polypeptides (or the nucleic acids which encode them). For example, it is common to recombinantly express polypeptides with one or more epitope tags, such as Myc (EQKLISEEDL) (SEQ ID NO: 3), Flag (DYKDDDDK) (SEQ ID NO: 4), KT3 tag sequence (KPPTPPPEPET) (SEQ ID NO: 5), T7 gene 10 (MASMTGGQQMGT) (SEQ ID NO: 6), poly-HIS, HA (YPYDVPDYA) (SEQ ID NO: 7), VSV-G (YTDIEMNRLGK) (SEQ ID NO: 8), HSV (QPELAPEDPED) (SEQ ID NO: 9), V5 (GKPIPNPLLGLDST) (SEQ ID NO: 10). This list is not meant to be limiting.

As used herein, the term "relating a signal to the presence or amount" of an analyte reflects this understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect Dashurin will also detect polypeptides related to the Dashurin sequence, so long as those polypeptides contain the epitope(s) necessary to bind to the antibody or antibodies used in the assay. The term "related marker" as used herein with regard to a biomarker such as Dashurin refers to one or more fragments of a particular marker or its biosynthetic parent that may be detected as a surrogate for the marker itself or as independent biomarkers. The term also refers to one or more polypeptides present in a biological sample that are derived from the Dashurin precursor complexed to additional species, such as binding proteins, receptors, heparin, lipids, sugars, *etc.*

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of a Dashurin assay of the present invention, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Dashurin Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (*e.g.,* fluorescent moieties, electrochemical labels, metal chelates, *etc.*) as well as molecules that may be indirectly detected by production of a detectable reaction product (*e.g.,* enzymes such as horseradish peroxidase, alkaline phosphatase, *etc.*) or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homo functional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, disclosed are also kits for the analysis of Dashurin, and optionally one or more other markers. The kit comprises reagents for the analysis of at least one test sample which comprise at least one antibody that binds Dashurin. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind Dashurin. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Dashurin Antibodies

The present invention also includes isolated antibodies which bind to one or more epitopes present in the Dashurin polypeptides of the present invention. The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind Dashurin. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ M$^{-1}$, and preferably between about $10^8$ M$^{-1}$ to about $10^9$ M$^{-1}$, about $10^9$ M$^{-1}$ to about $10^{10}$ M$^{-1}$, or about $10^{10}$ M$^{-1}$ to about $10^{12}$ M$^{-1}$.

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (*e.g.,* in sandwich assays) may interfere with one another sterically, *etc.,* assay performance of an antibody may be a more important measure than absolute affinity and specificity of an antibody.

Assay Correlations

The term "correlating" as used herein in reference to the use of biomarkers such as Dashurin refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection theory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, *etc.*) include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the Dashurin assay result of the present invention. These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alanine aminopeptidase (P15144); Alpha-1-acid glycoprotein 1 (P02763); Alpha-1-microglobulin (P02760); Albumin (P02768); Angiopoietin 1 (Ang1, Q15389); Angiotensinogenase (Renin, P00797); Annexin A2 (P07355); Basic fibroblast growth factor (bFGF, P09038); Beta-glucosidase (Q9H227); B-2-microglobulin (P61679); BMP-7 (P18075); Brain natriuretic peptide (proBNP, BNP-32, NTproBNP; P16860); Calbindin D28 (P05937); Calcium-binding protein Beta (P04271); Carbonic anhydrase (Q16790); Casein Kinase 2 (P12104); Cathepsin B (P07858); Ceruloplasmin (P00450); Clusterin (P10909); Collagen alpha-1(IV) chain (Q14051); Collagen alpha-2(IV) chain (P08572); Complement C3 (P01024); Cysteinerich protein (CYR61, 000622); Cytochrome C (P99999); EGF (Epidermal growth factor, P01133); Endothelin-1 (P05305); Erythropoietin (P01588); Exosomal Fetuin-A (P02765); Fatty acid-binding protein, liver (P07148); Fructose-1,6-biphosphatase (P09467); Granulocyte colony-stimulating factor (G-CSF, P09919); Granulocyte-macrophage colony-stimulating factor (P04141); GRO-alpha (CXCL1 P09341); GSTmu (Glutathione S-transferase Mu 1, P09488); Hepatocyte growth factor (P14210); Insulin-like growth factor I (P01343); Immunoglobulin G; Interferon gamma (P01308); Interleukin-1alpha (P01583); Interleukin-1be (P01584); Interleukin-2 (P60568); Interleukin-4 (P60568); Interleukin-10 (P22301); Interleukin-12p40 (P29460); Interleukin-16 (Q14005); L1 cell adhesion molecule (P32004); Lactate dehydrogenase (P00338); Meprin A-alpha subunit (Q16819); Meprin A-beta subunit (Q16820); Metalloproteinase inhibitor 2 (TIMP-2, P16035); Midkine (P21741); MIP2-alpha (CXCL2, P19875); MMP-2 (P08253); MMP-9 (P14780); Myeloperoxidase (P05164); Netrin-1 (O95631) Neutral endopeptidase (P08473); Osteoactivin (Glycoprotein (Transmembrane) nmb, A4D155); Osteopontin (P10451); Renal papillary antigen 1 (RNA1); Renal papillary antigen 2 (RPA2); Retinol binding protein (P09455); Podocin (Q9NP85); S100 calcium-binding protein A6 (P06703); S100 calcium-binding protein beta (P04271); (Serum Amyloid P Component (P02743); Sodium/Hydrogen exchanger isoform (NHE3, P48764); Spermidine/spermine N1-acetyltransferase (SAT) (P21673); TGF-Beta1 (P01137); Transferrin (P02787); Trefoil factor 3 (TFF3, Q07654); Thrombospondin-1 (P07996); Total protein; Tubulointerstitial nephritis antigen (Q9UJW2); Tumor necrosis factor-alpha (P01375); Uromodulin (Tamm-Horsfall protein, P07911); Vascular endothelial growth factor (VEGF, P15692); von Willebrand Factor (P04275).

For purposes of risk stratification, Adiponectin (Q15848); Alkaline phosphatase (P05186); Cystatin C (P01034); Delta subunit of FIFO ATPase (P30049); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, 000458); Isovaleryl-CoA dehydrogenase (IVD, P26440); I-TAC/CXCL11 (014625); Keratin 19 (P08727); TIMP-3 (P35625); Kim-1 (Hepatitis A virus cellular receptor 1, 043656; L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (014788); P8 protein (060356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61); RT1.B-1 (alpha) chain of the integral membrane protein (Q5Y7A8); Soluble tumor necrosis factor receptor superfamily member 1A (sTNFR-1, P19438); Soluble tumor necrosis factor receptor superfamily member 1B (sTNFR-II, P20333); and/or uPAR (Q03405) may be combined with the Dashurin assay result of the present invention.

Other clinical indicia which may be combined with the Dashurin assay result of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RNA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the Dashurin assay result are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal injury" and "acute renal failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 m$^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60\, mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

Selecting a Treatment Regimen

Once a diagnosis is obtained, the clinician can readily select a treatment regimen that is compatible with the diagnosis, such as initiating dialysis, withdrawing delivery of compounds that are known to be damaging to the kidney, kidney transplantation, *etc.* The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present invention may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, and are exemplary.

Example 1: Cloning of human Dashurin

Open reading frame 116 at chromosome 20 was found expressed at the kidney, in tumors and at various other sites as reported in myriads of gene arrays. When looking into further details, we found that this gene product might represent a secreted protein. After pre-treatment with DNAase I (15 min, room temperature), 1 $\mu$g of total human kidney RNA was subjected to reverse transcription (1 h, 40 °C) using Superscript II (Invitrogen, Carlsbad, CA, USA). cDNA synthesis was stopped by heating (15 min, 65 °C). After designing the primers (fw: 5'-ATGGTGGCGCCTGTGTG-3'; rev: 5'-TCAGGCTGGGGCTTGG-3'), Dashurin was PCR-amplified using the Expand™ High Fidelity PCR System (Boehringer Mannheim, Germany). The thermal cycling parameters were set to 35 cycles at 94°C (35s) for denaturation, 59.5°C (30s) for annealing and 72° (40s) for synthesis with a final extension period at 72 °C for 40 s.

Gel-purified PCR product was ligated into the pGEM®-T (Promega) easy vector using T4 DNA ligase (both from Promega) and incubated for 1 hour at room temperature. Subsequently the ligation reaction was used to transform MAX Efficiency® DH5$\alpha$ competent bacteria (Invitrogen) by the heat shock method. The transformation was plated onto LB/Amp/Xgal plates and white colonies were selected using colony PCR with the Dashurin specific primers. Plasmid purification was performed with Plasmid Mini Kit (Qiagen). In order to check the right insert purified plasmids were digested using restriction enzymes (EcoRI; XhoI; AvaI).

Example 2. Directional cloning of the human Dashurin coding sequence using Gateway technology

A new primer was designed including four nucleotides (CACC) at the 5' end of the forward primer for directional cloning of Dashurin-PCR product into pENTR/D-TOPO vector, (5'-CACCATGGTGGCGCCTGTGTG-3'). The gene of interest was produced using pGEM-T®easy-Dashurin as a template and the Expand™ High Fidelity PCR System (Boehringer Mannheim) (thermal cycling parameters were the same as described above). Cloning reaction: 3 μL PCR product, 1 μL pENTR/D-TOPO vector, 1 μL of salt solution and1 μL of $H_2O$ were mixed and incubated for 5 min at RT. The recombinant vector was transformed into MAX EFFICIENCY® DH5α Competent bacteria by the heat shock method, afterwards bacteria were incubated in S.O.C. medium for 30min. LB/Kan plates were used for selection of transformants. The correct orientation of transformants was analyzed by colony PCR and cultured for amplification in LB medium containing 50μg/ml kanamycin. Plasmids were isolated using Qiagen Plasmid Mini Kit. To confirm correct orientation of the insert, restriction analysis using XhoI and NotI was performed. For generating mammalian expression plasmids the clonase LR reaction was performed using pMSCV puro and for bacterial expression system pDESTTM 15 was used. The GATEWAY™ LR CLONASE™ reaction was performed as described in the manufacturer's manual by combining pENTR/D-TOPO-Dashurin-withSP and destination plasmid.

Example 3: Amplification of Dashurin emitting the signal peptide and its directional cloning using Gateway technology

A new forward primer (5'-CACCGCATCAGCCGGCCAAGAG-3') was designed to amplify Dashurin without the signal peptide. PCR was performed as described above using "Dashurin- pGEM®-T easy" as a template. The PCR product was gel purified and ligated into pENTR/D-TOPO vector for directional cloning. Competent bacteria were transformed and grown as described earlier. Colony PCR was performed to identify the colonies with the correct orientation, which were further cultured for amplification in 50μg/ml kanamycin containing LB medium. Plasmids were purified using Qiagen Plasmid Mini Kit.

Example 4: Transfer of Dashurin into the pEXP1-DEST and pcDNA-DEST53 vector

In order to create an expression plasmid, Dashurin without the signal peptide (Dashurin-woSP), was shifted from pENTR/D-TOPO into pEXP1-DEST performing a clonase reaction as described below: 1 μL (160ng) pENTR/D-TOPO-Dashurin, 1 μL (160ng) pEXP1-DEST, 6 μL TE buffer and 2 μL LR Clonase TM II were mixed and incubated for 1h at 25°C in a water bath. The same clonase reaction was performed to transfer the insert into pcDNA-DEST53 for obtaining mammalian expression Dashurin-encoding plasmids as an N-terminal fusion protein with GFP. LR recombination reaction was used to transform ONE SHOT® TOP10 (Invitrogen) chemically competent E.coli, subsequently plated onto LB/Amp agarose plates. Colonies were selected using restriction enzyme digests (XhoI, HindIII) analysis. The Dashurin-woSP insert encoding clones were purified using the QIAGEN Plasmid Mini Kit (Qiagen, Hilden, Germany) and sequenced.

Example 5: Cloning of Substance P-promoter luciferase reporter vector

The Pre-Pro-Tachykinin (PPTK) promoter was amplified using primers specific for human PPTK gene promoter (fw: 5'-CCCGCATCTCCCCTCAAGT-3', rev:5'-CAACCACCCAATCCCCCGAC-3') and high fidelity polymerase as described above using 61°C for annealing 72°C for synthesis and 94°C for denaturation each for 30sec with a final extension period of 7 min after the 35th cycle. The PCR product was ligated into SmaI site of pGL3 basic Luciferase Reporter Vector (Promega). Right orientation was verified by restriction enzyme digest and sequencing.

Example 6: Dashurin-woSP expression in BL21DE3

After insert verification, Dashurin-woSP-pEXP1 was used to transform E.coli bacteria (BL21DE3) by the heat shock method. Transformants were inoculated in 25ml YT/Amp medium and incubated over night at 37°C in a shaking incubator. After induction for 4h at 31°C using 100μM IPTG, bacteria were harvested by centrifugation at 3500rpm for 15min. The pellet was dissolved in BUGBUSTER® Protein Extraction Reagent (Novagen). BENSONASE® Nuclease (Novagen) and Lysozyme were added and incubated for 20min at room temperature according to the manufacturer's instructions. After centrifugation at 20 000g for 10min at 4°C the pellet was dissolved in 8M urea/BUGBUSTER® Protein Extraction Reagent (10:1, v/v). In order to accelerate the protein dissolving process, the pellet was sonicated with 10 strockes using the Labsonic U sonicator (B. Braun, Switzerland) at a setting of 250 power level. The supernatant from BUGBUSTER lysis and the dissolved pellet were combined and pre-cleared by spinning at 20 000g for 10min at 4°C before loading Ni-NTA His Bind Resin. Protein binding was carried out by rotating the tube for 30min at RT on a SB2 rotator (Stuart). After washing the Ni-NTA His6-Dashurin-woSP three times in 1xNi-NTA washing buffer (300 mM NaCl, 50 mM sodium phosphate buffer, 20 mM imidazole, pH 8.0), the recombinant protein was eluted using the 1x Ni-NTA elution buffer (300 mM NaCl, 250 mM sodium phosphate buffer, 50 mM imidazole, pH 8.0). Protein fractions were loaded onto SDS-PAGE (12% gels) and subsequently stained with Coomassie Blue in order to demonstrate the quantity of the eluted recombinant protein.

Example 7: Generating of the GST-Dashurin-woSP fusion protein

In order to clone the Dashurin-woSP in a GST gene fusion vector (pGEX-1λT) a new forward primer was designed including a BamHI site (5'-TCGGATCCGCATCAGCCGGCCAAGAG-3'). The PCR product (BamHI-Dashurin-woSP) was gel purified and ligated into pGEM®-T easy vector. After transformation of competent bacteria and plasmid purification (as described earlier), restriction enzyme digests (EcoRI, BamHI) was performed to confirm the insertion of BamHI-Dashurin-woSP into pGEM®-T easy. The gel purified insert was ligated into pGEX-1λT and was used to transform ONE SHOT® TOP10 chemically competent E.coli by the heat shock method. Growth of the colonies and plasmid purification was performed as already described. After sequence verification the newly cloned BamHI-Dashurin-woSP-pGEX-1λT was used for fusion protein expression in BL21DE3.

Example 8: Purification of GST-Dashurin-woSP fusion protein

Transformed BL21DE3 were inoculated in 25ml YT/Amp medium and incubated over night at 37°C in a shaking incubator. After the fusion protein expression was induced for 4h at 31 °C using 100μM IPTG, bacteria were harvested by centrifugation at 3500rpm for 15min. The pellet was suspended in 10ml 1xPBS and additionally sonicated in order to lyse the cells. Cell debris was pelleted by centrifugation at 20 000g for 10min at 4°C. Glutathione SepharoseTM 4B was added to the sonicated fusion protein supernatant. Protein binding was carried out by rotating the tube for 30min at 4°C on a SB2 rotator. The Glutathione Sepharose GST-Dashurin-woSP was washed twice with pre-cooled TPBS and twice with pre-cooled 1xPBS. Finally the pellet was resuspended in 300μL of 1xPBS. Ten units thrombin were added for liberating Dashurin-woSP protein from the fusion tag GST- Glutathione beads. Separation was performed by filtering the slurry through a mini column. In addition Coomassie Blue staining was performed to demonstrate the quantity of Dashurin-woSP protein.

Example 9: Generation of antibodies

His6-Dashurin-woSP fusion protein (200 μL), purified using Nickel affinity chromatography, was emulsified in complete Freund's adjuvant and subcutaneously injected into four weeks old Balb/C mice. The immunization was performed in three steps. Two additional immunizations with 200 μL in incomplete Freund's adjuvant were administered at two weeks interval. The collected serum was tested by parallel immunoblotting against the fusion protein and multiple human tissues.

Example 10: Generation of rabbit polyclonal antibody

Recombinant Dashurin was prepared by expressing His6-Dashurin-woSP and GST-Dashurin-woSP fusion proteins in E. coli. Both recombinant forms were used to immunize a 3-month-old rabbits. The purified recombinant Dashurin (300μL) emulsified in complete Freund's adjuvant was injected subcutaneously. Additinnal immunization with 200μL of purified protein in incomplete Freund's adjuvant was administered 21 days after the initial injection. The serum collected 6 weeks after the first immunization contained a high antibody activity against Dashurin when tested by Western blotting.

Example 11: Northern Blotting

Multiple-tissue Northern blots (BD Bioscience Clontech, USA) were used for hybridization with the nine exon spanning Dashurin-specific probe. According to the manufacturer's instructions (Ambion) hybridization was performed using ULTRAhyb solution. Briefly, after the pre-hybridization step for 2h at 42°C, blots were incubated overnight with the 32P-labelled probe in 10ml of ULTRAhyb solution and were then washed with NorthernMaxTM Low Stringency Wash solution # 1, subsequently washed with NorthernMaxTM High Strignency Wash Solution #2 (both from Ambion). Blots were overnight exposed to X-ray films at -80°C.
Variable quantities of Dashurin mRNA were expressed in different brain regions with highest quantity in medulla oblongata followed by cerebral cortex (Fig. 1).

Example 12: Western Blotting

Complementary to multiple tissue Northern analysis we investigated protein expression at various tumour samples. Protein aliquots were loaded onto 12% SDS-PAGE, which were run at 200 V at room temperature using Tris-glycine-SDS as a running buffer. A semidry blotting device was used to electrophoretically transfer the protein onto nitrocellulose. The blotted membrane was blocked with 2% skim milk/PBS for 30min. Antigen detection was performed (overnight, constant shaking at 4°C) with monoclonal anti-dashurin antibody. For detection of primary antibodies peroxidase-conjugated goat anti-mouse antibody was used. Each incubation step was followed by two washes with PBS containing 0.1% TWEEN® 20 surface active agent (ICI Americas) for 10 min. Antibody binding sites were visualised by BM chemiluminescence reagent. Electronic images were recorded using Lumi-Imager F1. Immunoblotting data confirmed the assumption that the mature protein representing the lower band devoid of the signal peptide whereas the upper band represents the pro-form of Dashurin (Fig. 2).

Complementary to multiple tissue Northern analysis we investigated protein expression at various tumour samples and surrounding normal human tissue. Immunoblotting data confirmed in endocrine tumors and pituitary adenomas that tumors show more expression then normal healthy tissue. In some tumors such as pituitary adenomas the pro-form was more abundant than the smaller variant. However, when renal clear cell carcinomas were compared with healthy normal tissue, much higher presence of Dashurin was found in healthy normal kidney tissue (Fig. 3).

Example 13: Quantitative real-time PCR (qRT-PCR)

Real-Time PCR was performed in duplicates using an ABI PRISM® 7000 Cycler (Applied Biosystems, Forster City, CA, USA). cDNA generated from different human tissues and tumors were used as template in TaqMan gene expression assays. The original cDNA transcript (2 $\mu$L) was used together with 2.5 $\mu$L of the 20x gene-specific 6-carboxyfluorescin (FAM)-carboxyltetramethyl-rhodamine(TAMRA)-labelled Assay-on-Demand C20orf116/Dashurin-specific probe (Applied Biosystems, Forster City, CA, USA) and 25 $\mu$L of the 2x PCR MasterMix (Roche Diagnostics) diluted to a final volume of 50 $\mu$L with H2O. Gene amplification was set into relation to GAPDH as endogenous control and calculated according to the $\Delta\Delta$CT method.

Example 14: Immunofluorescence

Human liver tissue, human kidney tissue, human placenta, cerebellum and tumor tissue such as pituitary adenoma, ovarian carcinoma, anaplastic thyroid carcinoma cryosections (4 $\mu$m) and cell lines such as myelomonocytic progenitor cells U937 and human embryonic kidney cell line Hek 293 cytopreparations were fixed in acetone for 5 min. The sections were overlaid with PBS and incubated with primary antibodies generated in mouse or in rabbit using recombinant protein. The incubation was either performed overnight at 4°C or reduced to 2h at room temperature, followed by 10 min washing with TPBS under constant stirring. For detection of the primary antibodies, goat anti-mouse FITC (Fluorescein isothiocyanate) conjugated antibodies or Alexa Fluor® 488 F(ab')2 fragment of goat anti-rabbit IgG (Invitrogen, USA) diluted 1: 200 in PBS and incubated for 45 min at room temperature. After a 10 min washing step, DAPI 4, 6-diamidino-2-phenylindole was applied in order to obtain DNA staining. After the final washing step in TPBS for 10 min under constant stirring, slides were mounted using VECTASHIELD® mounting medium (Vector Laboratories, Inc., Burlingame, CA, USA) and covered with a cover slip. A Zeiss Axiovert confocal microscope or the Leica Aristoplan was used to examine the slides. Pictures were recorded using Leica IM 500, version 1.20 and images were further arranged with Adobe Photoshop 6.

Within the kidneys we observed a varying staining of tubuli. Notably tubuli in proximity to glomerula stained highly together with presence in tublular lumen whereas most of tubules including the glomerulus showed faint immunostaining. Intraluminal presence in tubules depended on the kidney specimen, however, staining of primary urine within the Baumann's capsule was absent. Interestingly, confirming our previous data, expression of Dashurin was found in the nuclei of the epithelial cells in distinct tubuli. In order to test whether rapidly proliferating cells, such as Hek 293 show high expression of this protein we evaluated its location and found presence within the cell cytoplasm and nucleus. This stains were performed in cells grown in chamber slides, which were fixed *in topo* after washing off culture medium. Surprisingly, positive staining of nucleoli was also observed. Staining of pituitary tumour tissue revealed highly positive cytosol distribution as well as faint nuclear expression.

Example 15: Chamber slide culture

Hek 293 (5x105) cells were seeded into 2 chamber slides (Nunc Inter Med, Naperville, Illinois) and grown for 48 hours at 37°C, 5% CO2, 95% humidified atmosphere. The media was replaced with new complete culture medium containing 50 or 100nM Trichostatin A. After 24 hours culture medium was washed off using PBS and cells adherent to the glass were air dried for two hours. Cell fixation was carried out in - 20°C pre-cooled methanol for 15 minutes and primary antibody was applied after pre-wetting cells with PBS. Further staining procedure was carried out as described in the immunostaining.

Example 16: HepG2 cell fractionation

In order to answer the question of subcellular localization of the protein, we performed sucrose density gradient sedimentation of disrupted HepG2 hepatoma cells. HepG2 hepatoma cells were cultured in 150 x 25 mm Style polystyrene nonpyrogenic tissue culture dishes (Beckton Dickinson Labware Europe, France). At 75% confluence, culture dishes were washed twice with pre-cooled PBS and scraped into 2.5 ml of ice-cold homogenisation buffer (250 mM sucrose and 3 mM imidazole, ph 7.4) containing complete protease inhibitor cocktail (Roche Applied Science, Vienna, Austria). The cell suspension was then passed 5 times through 26-gauge needle and centrifuged at 2500 rpm for 10 minutes at 4°C in order to pellet unbroken cells and nuclei. The post-nuclear supernatant was loaded onto a discontinuous sucrose gradient (50% and 20% respectively) in 14 x 95 mm poly-allomer centrifuge tubes and centrifuged at 40 000 rpm for 3 hours at 4 °C in the SW40 TI motor of an ultracentrifuge (model L-80, Beckman instruments, Palo Alto, CA). Using a peristaltic pump, starting at the bottom 19 fractions were collected. The immunoblotting technique was used to analyze Dashurin content in the fractions.

19 fractions were collected and analyzed for presence of Dashurin. Surprisingly mature protein was solely found in light fractions corresponding to cytosolic proteins confirmed by sequential immunoblotting for Dashurin and β-actin. Scant quantities of the pro-Dashurin, was found in the membrane containing fractions. Furthermore, minimal quantity was found within the nucleus (Fig. 4). The *in silico* data predicted a PCI domain located at the C-terminus of the protein. This domain has been known to provide protein-protein interaction in several subunits of biologically highly active protein complexes. One of them is the proteasome. Therefore we looked for similar distribution of Dashurin together with the proteasome in disrupted Hep G2 cell cytosol by gel filtration chromatography. Dashurin distribution was detected in fractions 14-19 by immunoblot. Based on the fact that proteasomes occur in cell cytoplasm and in order to investigate whether Dashurin interacts through its PCI domain with proteasome subunits, Superose 6 column chromatographed cytosol fractions were analyzed for Rpt5 distribution. This represents a subunit of the 19S regulatory complex of the 26S proteasome. However, there was no evidence that Dashurin would co-segregate with the proteasome by appearing in the same fractions like Rpt5.

Example 17: Preparation of cytosol fractions from HepG2 cells

After culturing (as described earlier), HepG2 cells were homogenized in 4 vol. of 20mM Tris/HCl buffer, pH 7.5, containing 20% (v/v) glycerol, 1 mM 2-mercapthoethanol, 0.1mM EDTA and 5mM ATP and passed 5 times through 26-gauge needle. To pellet cell debris and nuclei, the homogenate was centrifuged for 1h at 95 000g. The resulting supernatant was fractionated by gel filtration using a Superose 6 column equilibrated with fluid phase 20mM Tris/HCl buffer, pH 7.5, containing 100mM NaCl and 10% (v/v) glycerol. This represents a method well characterized in previous literature for proteasome isolation and analysis (CITAT). See, e.g., Brooks et al., Biochem. J. 346: 155-161, 2000. Individual fractions as collected by a fraction collector were analysed by immunoblot for presence of various proteins such as proteasome subunits and Dashurin.

Example 18: FACS analysis

For cell-surface staining, U937 cells representing a myelomonocytic cell line and SH-SY5Y were chosen. After harvesting, cells were washed twice using pre-cooled PBS containing 0,1 % calf serum (wash solution) and incubated on ice for 30 min with mouse anti-human Dashurin-specific antibody including Beriglobin® P(Aventis Behring) for blocking Fc binding. As control pre-immunized mouse serum was employed. Subsequently, cells were washed twice and stained with FITC conjugated donkey anti-mouse antibody (30 min, 4°C). Following two washes with wash solution cell-surface fluorescence was measured using FACSCanto®II (BD Biosciences) and expression levels were demonstrated by DIVA software version 6.1.2 (BD Biosciences). For intracellular staining, after fixation in 100 μl paraformaldehyde for 10 min, cells were incubated in 80 μl Permeabilisation Medium (An der Grub, Bioresearch Gmbh, Austria) including mouse anti-human Dashurin-specific antibody for 30 min on ice. Donkey anti-mouse antibody-FITC was used for detection of the primary antibody together with BERIGLOBIN®P. The intracellular expression of Dashurin was assessed on a FACSCanto®II as described above.

A strong intracellular staining could be demonstrated for U 937 cells. In contrast, when SH-5Y5H neuroblastoma cells were investigated using the same method no difference between control and Dashurin antibody treated cells could be observed. In addition, cells were also looked at by surface staining. However, no antigen presence was found at the cell surface of U937 cells.

Example 19: Mammalian cell transfection

Neuroblastoma cells were cultured in RPMI medium supplemented with 10% FCS and streptomycin/penicillin in a 95% humidified incubator (5%CO2). Cells were passaged using trypsin the day before transfection and seeded to reach about 50% confluence 24 hrs after plating. Before transfection cells were washed twice with protein free RPMI and then exposed to transfection solution containing human substance P promoter-luciferase pGL3 and Dashurin-pMSCV puro (or pMSCV puro empty as a negative control) pre-treated with Lipofectamine Plus as described below. In Brief: 100$\mu$L protein free RPMI were used for diluting 600ng expression plasmid together with 1000ng luciferase reporter plasmid which was incubated for 15min with 8$\mu$L Plus TM Reagent (Invitrogen, Carlsbad, CA, USA). The pre-treated plasmid was then further incubated with (8$\mu$L/Petri dish) Lipofectamine TM Reagent (Invitrogen, Carlsbad, CA, USA) (which was pre-diluted in 100$\mu$L RPMI before adding to the plasmid solution). The lipofectamin plasmid mixture was added to pre-washed cells in a total volume of 1200$\mu$L RPMI solution and kept at the tissue culture incubator for 3hrs. Following the transfection period, the transfection solution was replaced using complete culture medium and incubated for 24h and replated for providing sufficient growth conditions. After 48h the luciferase activity was measured. Trnafected cells were rinsed with DPBS and lysed in 100$\mu$L/well luciferase lysis buffer. After collecting the cell lysate into Eppendorf tubes, 18$\mu$L from each cell lysate was used for measurement in luciferase assay substrate. The luciferase activity was assayed in a luminometer (Lumat LB 9507, Berthold technologies, Germany) and calculated as light units/cells.

Example 20: Dot Blot

Clean catch or catheter urine was centrifuged at 3000 rpm at the Alegra-XR (Backman/Coulter, USA) centrifuge for 10 min at 4°C and 100$\mu$L were pipetted into the dot blotting device (Schleicher & Schüler, Germany) while fluid was aspirated through a nitrocellulose membrane using a vacuum pump. The membrane was wetted with PBS after air-drying and incubated in blocking solution for 35min. Following a brief wash in PBS, the membrane was exposed to monospecific Dashurin rabbit antibody (1:5000) at RT for 2hr. After two washes in TPBS (10min) the second antibody (HRP conjugated goat anti-rabbit) was applied for 40min at RT (dilution 1:5000). Following two washes in TPBS (10min) the blot was developed with chemoluminescence reagent and evaluated by densitometric scanning using LumiAnalyst 3.1 software.

Example 21: Dashurin ELISA

REACTI-BIND™ (Thermo-Fisher) goat anti-rabbit IgG coated 96-well plates were incubated over night (16hr, at 4°C) for rabbit Ab capturing. The plate was then washed briefly with PBS and samples and standard were applied to the plate (100$\mu$L each) and incubated at room temperature for 2 h on a rotating shaker. After four washes with TPBS the second Ab was pipetted onto the plate (mouse anti Dashurin, dilution 1:1000 in PBS). Following an incubation period of 2h the plate was washed again 4 times using TPBS and the detection Ab (diluted 1:10000, HRP conjugated goat anti mouse) was applied after mixing with goat serum and human serum for blocking unsaturated plate surface. After an incubation period of 1.5h the plate was washed and the two component TMB substrate was added to the plate and reacted for 7min at RT. The reaction was stopped by adding 100$\mu$L 1M H3PO4 and read at the ELISA reader (Power wavex , Biotek instruments, Inc., USA) at 450 nm. Concentrations were calculated according to the standard curve which was obtained by measuring a serial dilution of recombinant Dashurin protein, diluted in insulin and c-peptide free human serum (Scantibodies laboratory, Inc., CA, USA).

Example 22: Urine Dashurin measurements in contrast patients

Acute kidney injury may be caused by administration of radiocontrast agents (also called contrast media) and other nephrotoxins such as cyclosporine, antibiotics including aminoglycosides and anticancer drugs. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

Plasma, urine and clinical data were collected from adult patients before and after receiving intravascular contrast media. To be enrolled in the study, each patient must meet all of the following inclusion criteria and none of the following exclusion criteria:

Inclusion Criteria
males and females 18 years of age or older;
undergoing a radiographic / angiographic procedure (such as a CT scan or coronary intervention) involving the intravascular administration of contrast media;
expected to be hospitalized for at least 48 hours after contrast administration.
able and willing to provide written informed consent for study participation and to comply with all study procedures.

Exclusion Criteria

renal transplant recipients;

acutely worsening renal function prior to the contrast procedure;

already receiving dialysis (either acute or chronic) or in imminent need of dialysis at enrollment;

expected to undergo a major surgical procedure (such as involving cardiopulmonary bypass) or an additional imaging procedure with contrast media with significant risk for further renal insult within the 48 hrs following contrast administration;

participation in an interventional clinical study with an experimental therapy within the previous 30 days;

known infection with human immunodeficiency virus (HIV) or a hepatitis virus.

Immediately prior to the first contrast administration (and after any pre-procedure hydration), an EDTA anti-coagulated blood sample (10 mL) and a urine sample (10 mL) were collected from each patient. Blood and urine samples were then collected at 2 ($\pm$0.5), 4 ($\pm$0.5), 8 ($\pm$1), 24 ($\pm$2) and 48 ($\pm$2) hrs following the last administration of contrast media during the index contrast procedure. Blood was collected via direct venipuncture or via other available venous access, such as an existing femoral sheath, central venous line, peripheral intravenous line or hep-lock.

Serum creatinine was assessed immediately prior to the first contrast administration (after any pre-procedure hydration) and at 2 ($\pm$0.5), 4 ($\pm$0.5), 8 ($\pm$1), 24 ($\pm$2) and 48 ($\pm$2) hrs following the last administration of contrast (ideally at the same time as the study samples are obtained). In addition, each patient's status was evaluated through day 7 with regard to additional serum creatinine measurements, a need for dialysis, hospitalization status, and adverse clinical outcomes (including mortality).

Prior to contrast administration, each patient was assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 m$^2$ = 2 points, 20-40 mL/min/1.73 m$^2$ = 4 points, < 20 mL/min/1.73 m$^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1%, risk of dialysis - 1.09%; >16 total points = risk of CIN - 57.3%, risk of dialysis - 12.8%.

Urine Dashurin levels were obtained for 31 enrolled patients receiving contrast and, for comparison, 17 normal healthy donors. In the patient group, mean Dashurin levels were 136 (standard deviation 82), which was significantly higher (p=0.023) than in normals (85; standard deviation 35).

Example 23. Urine dashurin measurement in ICU patients

Patients from the intensive care unit (ICU) were enrolled in the following study. Each patient was classified by kidney status as non-injury (0), risk of injury (R), injury (I), and failure (F) according to the maximum stage reached within 7 days of enrollment as determined by the RIFLE criteria. EDTA anti-coagulated blood samples (10 mL) and a urine samples (25-30 mL) were collected from each patient at enrollment, 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Dashurin was measured by standard immunoassay methods using commercially available assay reagents in the urine samples and the plasma component of the blood samples collected.

Two cohorts were defined as described in the introduction to each of the following tables. In the following tables, the time "prior max stage" represents the time at which a sample is collected, relative to the time a particular patient reaches the lowest disease stage as defined for that cohort, binned into three groups which are +/- 12 hours. For example, "24 hr prior" which uses 0 vs R, I, F as the two cohorts would mean 24 hr (+/- 12 hours) prior to reaching stage R (or I if no sample at R, or F if no sample at R or I).

A receiver operating characteristic (ROC) curve was generated for Dashurin and the area under each ROC curve (AUC) was determined. Patients in Cohort 2 were also separated according to the reason for adjudication to cohort 2 as being based on serum creatinine measurements (sCr), being based on urine output (UO), or being based on either serum creatinine measurements or urine output. Using the same example discussed above (0 vs R, I, F), for those patients adjudicated to stage R, I, or F on the basis of serum creatinine measurements alone, the stage 0 cohort may have included patients adjudicated to stage R, I, or F on the basis of urine output; for those patients adjudicated to stage R, I, or F on the basis of urine output alone, the stage 0 cohort may have included patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, in the data for patients adjudicated on the basis of serum creatinine measurements or urine output, the adjudication method which yielded the most severe RIFLE stage was used.

The ability to distinguish cohort 1 from Cohort 2 was determined using ROC analysis. SE is the standard error of the AUC, n is the number of sample or individual patients ("pts," as indicated). Standard errors were calculated as described in Hanley, J. A., and McNeil, B.J., The meaning and use of the area under a receiver operating characteristic (ROC) curve. Radiology (1982) 143: 29-36; p values were calculated with a two-tailed Z-test, and are reported as p<0.05 in tables 1-4 and p<0.10 in tables 5-6. An AUC < 0.5 is indicative of a negative going marker for the comparison, and an AUC > 0.5 is indicative of a positive going marker for the comparison.

Various dashurin threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

Table 1: Comparison of marker levels in urine samples collected for Cohort 1 (patients that did not progress beyond RIFLE stage 0,R, or I) and in urine samples collected from subjects at 0, 24 hours, and 48 hours prior to reaching stage F in Cohort 2.

| sCr or UO | 24hr prior to AKI stage | |
|---|---|---|
| | Cohort 1 | Cohort 2 |
| Median | 0.49 | 5.9 |
| Average | 33 | 27 |
| Stdev | 180 | 56 |
| p(t-test) | | 0.90 |
| Min | 0.054 | 0.085 |
| Max | 2400 | 190 |
| n (Samp) | 432 | 13 |
| n (Patient) | 217 | 13 |

| | 24hr prior to AKI stage |
|---|---|
| | sCr or UO |
| AUC | 0.67 |
| SE | 0.083 |
| p | 0.037 |
| nCohort 1 | 432 |
| nCohort 2 | 13 |
| Cutoff 1 | 0.054 |
| Sens 1 | 100% |
| Spec 1 | 1% |
| Cutoff 2 | 0.054 |
| Sens 2 | 100% |
| Spec 2 | 1% |
| Cutoff 3 | 0.054 |
| Sens 3 | 100% |
| Spec 3 | 1% |
| Cutoff 4 | 2.4 |
| Sens 4 | 69% |
| Spec 4 | 70% |
| Cutoff 5 | 4.1 |

(continued)

| | |
|---|---|
| Sens 5 | 54% |
| Spec 5 | 80% |
| Cutoff 6 | 9.7 |
| Sens 6 | 38% |
| Spec 6 | 90% |
| OR Quart 2 | 0 |
| p Value | na |
| 95% CI of | na |
| OR Quart2 | na |
| OR Quart 3 | 0.49 |
| p Value | 0.42 |
| 95% CI of | 0.088 |
| OR Quart3 | 2.7 |
| OR Quart 4 | 1.8 |
| p Value | 0.37 |
| 95% CI of | 0.51 |
| OR Quart4 | 6.3 |

Table 2: Comparison of marker levels in urine samples collected from Cohort 1 (patients that did not progress beyond RIFLE stage 0, R, or I) and in urine samples

collected from Cohort 2 (subjects who progress to RIFLE stage F) at 0, 24 hours, and 48 hours prior to the subject reaching RIFLE stage I.

| sCr or UO | 0hr prior to AKI stage | | 24hr prior to AKI stage | |
|---|---|---|---|---|
| | Cohort 1 | Cohort 2 | Cohort 1 | Cohort 2 |
| Median | 0.49 | 3.4 | 0.49 | 5.6 |
| Average | 33 | 94 | 33 | 98 |
| Stdev | 180 | 270 | 180 | 280 |
| p(t-test) | | 0.29 | | 0.19 |
| Min | 0.054 | 0.085 | 0.054 | 0.085 |
| Max | 2400 | 860 | 2400 | 1000 |
| n (Samp) | 432 | 10 | 432 | 14 |
| n (Patient) | 217 | 10 | 217 | 14 |

| UO only | 24hr prior to AKI stage | |
|---|---|---|
| | Cohort 1 | Cohort 2 |
| Median | 0.42 | 7.5 |
| Average | 39 | 110 |
| Stdev | 190 | 300 |
| p(t-test) | | 0.20 |
| Min | 0.054 | 0.085 |
| Max | 2400 | 1000 |
| n (Samp) | 355 | 12 |
| n (Patient) | 178 | 12 |

| | 0hr prior to AKI stage | | 24hr prior to AKI stage | |
|---|---|---|---|---|
| | sCr or UO | UO only | sCr or UO | UO only |
| AUC | 0.70 | nd | 0.66 | 0.69 |
| SE | 0.094 | nd | 0.081 | 0.086 |
| p | 0.037 | nd | 0.052 | 0.030 |
| nCohort 1 | 432 | nd | 432 | 355 |
| nCohort 2 | 10 | nd | 14 | 12 |
| Cutoff 1 | 2.4 | nd | 0.054 | 0.054 |
| Sens 1 | 70% | nd | 100% | 100% |
| Spec 1 | 70% | nd | 1% | 1% |
| Cutoff 2 | 0.78 | nd | 0.054 | 0.054 |
| Sens 2 | 80% | nd | 100% | 100% |
| Spec 2 | 55% | nd | 1% | 1% |
| Cutoff 3 | 0.054 | nd | 0.054 | 0.054 |
| Sens 3 | 100% | nd | 100% | 100% |
| Spec 3 | 1% | nd | 1% | 1% |
| Cutoff 4 | 2.4 | nd | 2.4 | 2.3 |
| Sens 4 | 70% | nd | 64% | 67% |
| Spec 4 | 70% | nd | 70% | 70% |
| Cutoff 5 | 4.1 | nd | 4.1 | 4.1 |
| Sens 5 | 50% | nd | 57% | 67% |

| | 0hr prior to AKI stage | | 24hr prior to AKI stage | |
| --- | --- | --- | --- | --- |
| | sCr or UO | UO only | sCr or UO | UO only |
| Spec 5 | 80% | nd | 80% | 80% |
| Cutoff 6 | 9.7 | nd | 9.7 | 10 |
| Sens 6 | 30% | nd | 36% | 42% |
| Spec 6 | 90% | nd | 90% | 90% |
| OR Quart 2 | 0 | nd | 0 | 0 |
| p Value | na | nd | na | na |
| 95% CI of | na | nd | na | na |
| OR Quart2 | na | nd | na | na |
| OR Quart 3 | 1.5 | nd | 0.19 | 0 |
| p Value | 0.65 | nd | 0.14 | na |
| 95% CI of | 0.25 | nd | 0.022 | na |
| OR Quart3 | 9.2 | nd | 1.7 | na |
| OR Quart 4 | 2.5 | nd | 1.6 | 2.1 |
| p Value | 0.27 | nd | 0.40 | 0.25 |
| 95% CI of | 0.48 | nd | 0.52 | 0.60 |
| OR Quart4 | 13 | nd | 5.1 | 7.1 |

| | Cohort 1 | Cohort 2 | Cohort 1 | Cohort 2 |
|---|---|---|---|---|
| Median | 1.18 | 9.86 | 1.18 | 10.9 |
| Average | 44.2 | 209 | 44.2 | 190 |
| Stdev | 256 | 423 | 256 | 410 |
| p(t-test) | | 0.034 | | 0.069 |
| Min | 0.0851 | 0.0851 | 0.0851 | 0.0851 |
| Max | 2360 | 1250 | 2360 | 1250 |
| n (Samp) | 99 | 16 | 99 | 14 |
| n (Patient) | 99 | 16 | 99 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.76 | 0.70 | 0.81 | 0.73 | nd | 0.81 | 0.78 | nd | nd |
| SE | 0.062 | 0.090 | 0.067 | 0.071 | nd | 0.073 | 0.089 | nd | nd |
| p | 2.4E-5 | 0.023 | 2.9E-6 | 9.2E-4 | nd | 2.4E-5 | 0.0018 | nd | nd |
| nCohort 1 | 118 | 182 | 99 | 118 | nd | 99 | 118 | nd | nd |
| nCohort 2 | 22 | 11 | 16 | 18 | nd | 14 | 10 | nd | nd |
| Cutoff 1 | 5.17 | 2.36 | 6.43 | 2.64 | nd | 8.23 | 5.17 | nd | nd |
| Sens 1 | 73% | 73% | 75% | 72% | nd | 71% | 70% | nd | nd |
| Spec 1 | 79% | 57% | 83% | 64% | nd | 88% | 79% | nd | nd |
| Cutoff 2 | 2.36 | 1.79 | 5.99 | 1.78 | nd | 5.17 | 1.78 | nd | nd |
| Sens 2 | 82% | 82% | 81% | 83% | nd | 86% | 90% | nd | nd |
| Spec 2 | 58% | 52% | 83% | 54% | nd | 81% | 54% | nd | nd |
| Cutoff 3 | 0 | 0 | 0 | 0 | nd | 0 | 1.78 | nd | nd |
| Sens 3 | 100% | 100% | 100% | 100% | nd | 100% | 90% | nd | nd |
| Spec 3 | 0% | 0% | 0% | 0% | nd | 0% | 54% | nd | nd |
| Cutoff 4 | 3.64 | 3.76 | 3.74 | 3.64 | nd | 3.74 | 3.64 | nd | nd |
| Sens 4 | 77% | 64% | 88% | 67% | nd | 86% | 70% | nd | nd |
| Spec 4 | 70% | 70% | 71% | 70% | nd | 71% | 70% | nd | nd |
| Cutoff 5 | 5.99 | 7.07 | 5.17 | 5.99 | nd | 5.17 | 5.99 | nd | nd |
| Sens 5 | 64% | 45% | 88% | 61% | nd | 86% | 60% | nd | nd |
| Spec 5 | 81% | 80% | 81% | 81% | nd | 81% | 81% | nd | nd |
| Cutoff 6 | 11.7 | 11.7 | 11.0 | 11.7 | nd | 11.0 | 11.7 | nd | nd |
| Sens 6 | 32% | 36% | 44% | 33% | nd | 50% | 30% | nd | nd |
| Spec 6 | 91% | 90% | 91% | 91% | nd | 91% | 91% | nd | nd |
| OR Quart 2 | >4.5 | 1.0 | 0 | >4.5 | nd | 0 | >1.0 | nd | nd |
| p Value | <0.19 | 1.0 | na | <0.19 | nd | na | <0.98 | nd | nd |
| 95% CI of | >0.48 | 0.061 | na | >0.48 | nd | na | >0.062 | nd | nd |
| OR Quart2 | na | 16 | na | na | nd | na | na | nd | nd |
| OR Quart 3 | >5.8 | 3.1 | 0.96 | >3.3 | nd | 0.48 | >2.1 | nd | nd |
| p Value | <0.12 | 0.33 | 0.97 | <0.31 | nd | 0.56 | <0.54 | nd | nd |
| 95% CI of | >0.65 | 0.31 | 0.13 | >0.32 | nd | 0.041 | >0.18 | nd | nd |
| OR Quart3 | na | 31 | 7.4 | na | nd | 5.6 | na | nd | nd |
| OR Quart 4 | >21 | 6.6 | 9.2 | >16 | nd | 7.9 | >9.0 | nd | nd |
| p Value | <0.0047 | 0.087 | 0.0072 | <0.0097 | nd | 0.012 | <0.047 | nd | nd |
| 95% CI of | >2.5 | 0.76 | 1.8 | >2.0 | nd | 1.6 | >1.0 | nd | nd |
| OR Quart4 | na | 57 | 46 | na | nd | 40 | na | nd | nd |

Table 4: Comparison of marker levels in urine samples collected for Cohort 1 (patients that did not progress beyond RIFLE stage 0 or R) and in urine samples collected from subjects at 0, 24 hours, and 48 hours prior to reaching stage I or F in Cohort 2.

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.591 | 0.688 | 0.591 | 1.83 | 0.591 | 1.58 |
| Average | 20.6 | 122 | 20.6 | 112 | 20.6 | 45.2 |
| Stdev | 149 | 337 | 149 | 297 | 149 | 182 |
| p(t-test) | | 0.0034 | | 0.0018 | | 0.50 |
| Min | 0.0539 | 0.0851 | 0.0539 | 0.0851 | 0.0539 | 0.0851 |
| Max | 2360 | 1250 | 2360 | 1040 | 2360 | 773 |
| n (Samp) | 339 | 26 | 339 | 37 | 339 | 18 |
| n (Patient) | 183 | 26 | 183 | 37 | 183 | 18 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.700 | 2.70 | 0.700 | 1.35 |
| Average | nd | nd | 34.5 | 33.8 | 34.5 | 146 |
| Stdev | nd | nd | 180 | 66.9 | 180 | 415 |
| p(t-test) | nd | nd | | 0.99 | | 0.077 |
| Min | nd | nd | 0.0539 | 0.0851 | 0.0539 | 0.0851 |
| Max | nd | nd | 2360 | 192 | 2360 | 1250 |
| n (Samp) | nd | nd | 416 | 9 | 416 | 9 |
| n (Patient) | nd | nd | 214 | 9 | 214 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.372 | 0.688 | 0.372 | 1.47 | 0.372 | 2.12 |
| Average | 23.4 | 122 | 23.4 | 122 | 23.4 | 50.8 |
| Stdev | 163 | 337 | 163 | 309 | 163 | 193 |
| p(t-test) | | 0.0096 | | 0.0035 | | 0.52 |
| Min | 0.0539 | 0.0851 | 0.0539 | 0.0851 | 0.0539 | 0.0851 |
| Max | 2360 | 1250 | 2360 | 1040 | 2360 | 773 |
| n (Samp) | 278 | 26 | 278 | 34 | 278 | 16 |
| n (Patient) | 150 | 26 | 150 | 34 | 150 | 16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | nd | 0.56 | 0.59 | 0.66 | 0.60 | 0.54 | 0.57 | 0.56 |
| SE | 0.060 | nd | 0.061 | 0.051 | 0.10 | 0.054 | 0.071 | 0.10 | 0.076 |
| p | 0.52 | nd | 0.33 | 0.095 | 0.11 | 0.057 | 0.53 | 0.51 | 0.40 |
| nCohort 1 | 339 | nd | 278 | 339 | 416 | 278 | 339 | 416 | 278 |
| nCohort 2 | 26 | nd | 26 | 37 | 9 | 34 | 18 | 9 | 16 |
| Cutoff 1 | 0.0539 | nd | 0.0539 | 0.0539 | 1.89 | 0.0539 | 0.0539 | 0.0539 | 0.0539 |
| Sens 1 | 100% | nd | 100% | 100% | 78% | 100% | 100% | 100% | 100% |
| Spec 1 | 1% | nd | 1% | 1% | 64% | 1% | 1% | 1% | 1% |
| Cutoff 2 | 0.0539 | nd | 0.0539 | 0.0539 | 0.0539 | 0.0539 | 0.0539 | 0.0539 | 0.0539 |
| Sens 2 | 100% | nd | 100% | 100% | 100% | 100% | 100% | 100% | 100% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 2 | 1% | nd | 1% | 1% | 1% | 1% | 1% | 1% | 1% |
| Cutoff 3 | 0.0539 | nd | 0.0539 | 0.0539 | 0.0539 | 0.0539 | 0.0539 | 0.0539 | 0.0539 |
| Sens 3 | 100% | nd | 100% | 100% | 100% | 100% | 100% | 100% | 100% |
| Spec 3 | 1% | nd | 1% | 1% | 1% | 1% | 1% | 1% | 1% |
| Cutoff 4 | 2.61 | nd | 2.21 | 2.61 | 2.80 | 2.21 | 2.61 | 2.80 | 2.21 |
| Sens 4 | 31% | nd | 35% | 38% | 44% | 47% | 33% | 33% | 50% |
| Spec 4 | 70% | nd | 70% | 70% | 70% | 70% | 70% | 70% | 70% |
| Cutoff 5 | 4.15 | nd | 4.14 | 4.15 | 4.64 | 4.14 | 4.15 | 4.64 | 4.14 |
| Sens 5 | 27% | nd | 27% | 35% | 33% | 35% | 28% | 33% | 31% |
| Spec 5 | 81% | nd | 80% | 81% | 80% | 80% | 81% | 80% | 80% |
| Cutoff 6 | 8.42 | nd | 8.32 | 8.42 | 10.6 | 8.32 | 8.42 | 10.6 | 8.32 |
| Sens 6 | 19% | nd | 19% | 27% | 22% | 29% | 11% | 22% | 12% |
| Spec 6 | 90% | nd | 90% | 90% | 90% | 90% | 90% | 90% | 90% |
| OR Quart 2 | 0.090 | nd | 0.088 | 0.14 | >2.0 | 0.071 | >8.8 | >4.2 | >7.6 |
| p Value | 0.023 | nd | 0.022 | 0.0099 | <0.56 | 0.012 | <0.043 | <0.21 | <0.061 |
| 95% CI of | 0.011 | nd | 0.011 | 0.030 | >0.18 | 0.0090 | >1.1 | >0.46 | >0.91 |
| OR Quart2 | 0.72 | nd | 0.71 | 0.62 | na | 0.56 | na | na | na |
| OR Quart 3 | 0.68 | nd | 0.57 | 0.66 | >3.1 | 0.72 | >4.2 | >2.0 | >3.1 |
| p Value | 0.45 | nd | 0.30 | 0.37 | <0.33 | 0.48 | <0.20 | <0.56 | <0.33 |
| 95% CI of | 0.25 | nd | 0.19 | 0.27 | >0.32 | 0.28 | >0.46 | >0.18 | >0.32 |
| OR Quart3 | 1.9 | nd | 1.6 | 1.6 | na | 1.8 | na | na | na |
| OR Quart 4 | 0.77 | nd | 0.89 | 1.0 | >4.1 | 1.0 | >6.4 | >3.1 | >6.4 |
| p Value | 0.60 | nd | 0.81 | 1.0 | <0.21 | 1.0 | <0.090 | <0.34 | <0.088 |
| 95% CI of | 0.29 | nd | 0.34 | 0.44 | >0.45 | 0.42 | >0.75 | >0.31 | >0.76 |
| OR Quart4 | 2.1 | nd | 2.3 | 2.3 | na | 2.4 | na | na | na |

Publications:

Asano, K., Kinzy, T. G., Merrick, W. C., and Hershey, J. W. (1997a). Conservation and diversity of eukaryotic translation initiation factor eIF3. J Biol Chem 272, 1101-1109.

Asano, K., Merrick, W. C., and Hershey, J. W. (1997b). The translation initiation factor eIF3-p48 subunit is encoded by int-6, a site of frequent integration by the mouse mammary tumor virus genome. J Biol Chem 272, 23477-23480.

Asano, K., Vornlocher, H. P., Richter-Cook, N. J., Merrick, W. C., Hinnebusch, A. G., and Hershey, J. W. (1997c). Structure of cDNAs encoding human eukaryotic initiation factor 3 subunits. Possible roles in RNA binding and macromolecular assembly. J Biol Chem 272, 27042-27052.

Block, K. L., Vornlocher, H. P., and Hershey, J. W. (1998). Characterization of cDNAs encoding the p44 and p35 subunits of human translation initiation factor eIF3. J Biol Chem 273, 31901-31908.

Casals, G., Filella, X., Auge, J. M., and Bedini, J. L. (2008). Impact of ultrasensitive cardiac troponin I dynamic changes in the new universal definition of myocardial infarction. Am J Clin Pathol 130, 964-968.

Daibata, M., Taguchi, T., and Taguchi, H. (2002). A novel t(16;20)(q22;p13) in polycythemia vera. Cancer Genet Cytogenet 137, 29-32.

Ewing, R. M., Chu, P., Elisma, F., Li, H., Taylor, P., Climie, S., McBroom-Cerajewski, L., Robinson, M. D., O'Connor, L., Li, M., et al. (2007). Large-scale mapping of human protein-protein interactions by mass spectrometry. Mol Syst Biol 3, 89.

Hofmann, K., and Bucher, P. (1998). The PCI domain: a common theme in three multiprotein complexes. Trends Biochem Sci 23, 204-205.

Johnson, K. R., Merrick, W. C., Zoll, W. L., and Zhu, Y. (1997). Identification of cDNA clones for the large subunit of eukaryotic translation initiation factor 3. Comparison of homologues from human, Nicotiana tabacum, Caenorhabditis elegans, and Saccharomyces cerevisiae. J Biol Chem 272, 7106-7113.

Kurian, S. M., Flechner, S. M., Kaouk, J., Modlin, C., Goldfarb, D., Cook, D. J., Head, S., and Salomon, D. R. (2005). Laparoscopic donor nephrectomy gene expression profiling reveals upregulation of stress and ischemia associated genes compared to control kidneys. Transplantation 80, 1067-1071.

Lenburg, M. E., Liou, L. S., Gerry, N. P., Frampton, G. M., Cohen, H. T., and Christman, M. F. (2003). Previously unidentified changes in renal cell carcinoma gene expression identified by parametric analysis of microarray data. BMC Cancer 3, 31.

Methot, N., Rom, E., Olsen, H., and Sonenberg, N. (1997). The human homologue of the yeast Prt1 protein is an integral part of the eukaryotic initiation factor 3 complex and interacts with p170. J Biol Chem 272, 1110-1116.

Pei, J., Balsara, B. R., Li, W., Litwin, S., Gabrielson, E., Feder, M., Jen, J., and Testa, J. R. (2001). Genomic imbalances in human lung adenocarcinomas and squamous cell carcinomas. Genes Chromosomes Cancer 31, 282-287.

Rapoport, T. A. (2007). Protein translocation across the eukaryotic endoplasmic reticulum and bacterial plasma membranes. Nature 450, 663-669.

Stitziel, N. O., Mar, B. G., Liang, J., and Westbrook, C. A. (2004). Membrane-associated and secreted genes in breast cancer. Cancer Res 64, 8682-8687.

von Heijne, G. (1985). Signal sequences. The limits of variation. J Mol Biol 184, 99-105.

Xue, Y., Yu, F., Xin, Y., Lu, D., Zou, Z., Guo, Y., and Xie, X. (1997). t(8;20)(q22;p13): a novel variant translocation of t(8;21) in acute myeloblastic leukaemia. Br J Haematol 98, 733-735.

Yamamoto, K., Hamaguchi, H., Nagata, K., Kobayashi, M., Takashima, T., and Taniwaki, M. (1998). A new complex translocation (15;20;17)(q22;p13;q21) in acute promyelocytic leukemia. Cancer Genet Cytogenet 101, 89-94.

The examples provided herein are representative of preferred embodiments, and are exemplary. Thus, for example, in each instance herein any of the terms "comprising", "consisting essentially of" and "consisting of" may be replaced with either of the other two terms.

**Claims**

1. A method for evaluating renal status in a subject, comprising:

    performing an assay method configured to detect one or more biomarkers comprising Dashurin on a body fluid sample obtained from the subject to provide an assay result,
    wherein the assay generates a detectable signal indicative of the presence or amount of a physiologically relevant concentration of Dashurin of SEQ ID NO: 1 and/or immunoreactive fragments thereof; and
    correlating the assay result to the renal status of the subject.

2. A method according to claim 1, wherein said correlation step comprises

    (i) correlating the assay result to one or more of risk stratification, diagnosis, staging, classifying and monitoring of the renal status of the subject, or
    (ii) assigning a likelihood of one or more future changes in renal status to the subject based on the assay result.

3. A method according to claim 2, wherein said one or more future changes in renal status comprise one or more of a future injury to renal function, future reduced renal function, future improvement in renal function, and future acute renal failure (ARF).

4. A method according to claim 2 or 3, wherein the likelihood of one or more future changes in renal status is that an event of interest is more or less likely to occur within a period of 72 hours of the time at which the body fluid sample is obtained from the subject.

5. A method according to claim 1, wherein the subject is not in acute renal failure.

6. A method according to claim 1, wherein the subject has not experienced a 1.5-fold or greater increase in serum creatinine over a baseline value determined prior to the time at which the body fluid sample is obtained or, wherein the subject has a urine output of at least 0.5 ml/kg/hr over the 12 hours preceding the time at which the body fluid sample is obtained.

7. A method according to claim 1, wherein the subject has not experienced an increase of 0.3 mg/dL or greater in serum creatinine over a baseline value determined prior to the time at which the body fluid sample is obtained, or wherein the subject (i) has not experienced a 1.5-fold or greater increase in serum creatinine over a baseline value determined prior to the time at which the body fluid sample is obtained, (ii) has a urine output of at least 0.5 ml/kg/hr over the 12 hours preceding the time at which the body fluid sample is obtained, and (iii) has not experienced an increase of 0.3 mg/dL or greater in serum creatinine over a baseline value determined prior to the time at which the body fluid sample is obtained.

8. A method according to claim 1, wherein the subject is in RIFLE stage 0 or R.

9. A method according to claim 8, wherein

(i) the subject is in RIFLE stage 0, and said correlating step comprises assigning a likelihood that the subject will reach RIFLE stage R or I within 72 hours, or
(ii) wherein the subject is in RIFLE stage 0 or R, and said correlating step comprises assigning a likelihood that the subject will reach RIFLE stage I or F within 72 hours or within 48 hours or within 24 hours.

10. A method according to claim 1, wherein the subject is in RIFLE stage I, and said correlating step comprises assigning a likelihood that the subject will reach RIFLE stage F within 72 hours, or within 48 hours or within 24 hours.

11. A method according to claim 1, wherein said assay result(s) comprise a measured urine or plasma concentration of Dashurin and said correlation step comprises comparing said measure concentration to a threshold concentration, and
assigning an increased likelihood of progressing to a worsening RIFLE stage to the subject, relative to the subject's current RIFLE stage, when the measured concentration is above the threshold, or assigning a decreased likelihood of progressing to a worsening RIFLE stage to the subject, relative to the subject's current RIFLE stage, when the measured concentration is below the threshold, or
wherein said assay result(s) comprise a measured urine or plasma concentration of Dashurin and said correlation step comprises comparing said measure concentration to a threshold concentration, and
assigning an increased likelihood of progressing to a need for renal replacement therapy to the subject when the measured concentration is above the threshold, or assigning a decreased likelihood of progressing to a need for renal replacement therapy when the measured concentration is below the threshold, or
wherein said assay result(s) comprise a measured urine or plasma concentration of Dashurin and said correlation step comprises comparing said measure concentration to a threshold concentration, and
assigning an increased likelihood of progressing to a worsening RIFLE stage to the subject, relative to the subject's current RIFLE stage, when the measured concentration is above the threshold, or assigning a decreased likelihood of progressing to a worsening RIFLE stage to the subject, relative to the subject's current RIFLE stage, when the measured concentration is below the threshold.

12. A method according to claim 5, wherein said assay result(s) comprise a measured urine or plasma concentration of Dashurin and said correlation step comprises comparing said measure concentration to a threshold concentration, and
assigning an increased likelihood of progressing to acute renal failure when the measured concentration is above the threshold, or assigning a decreased likelihood of progressing to acute renal failure to the subject when the measured concentration is below the threshold.

13. A method according to claim 9, wherein said assay result(s) comprise a measured urine or plasma concentration of Dashurin and said correlation step comprises comparing said measured concentration to a threshold concentration, and assigning an increased likelihood of progressing to RIFLE stage R, I or F to the subject, when the measured concentration is above the threshold, or assigning a decreased likelihood of progressing to RIFLE stage R, I or F to the subject when the measured concentration is below the threshold.

14. A method according to claim 1, wherein the subject is selected for evaluation of renal status based on

(i) the pre-existence in the subject of one or more known risk factors for prerenal, intrinsic renal, or postrenal ARF, or
(ii) an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

15. The use of Dashurin of SEQ ID NO:1 and/or immunoreactive fragments thereof for the diagnosis, risk stratification, prognosis, classifying and monitoring of renal status of a subject not receiving renal replacement therapy, for the diagnosis, risk stratification, prognosis, classifying and monitoring of renal status of a subject not in acute renal failure, or for assigning an increased likelihood of progressing to a worsening RIFLE stage to a subject, relative to the subject's current RIFLE stage.

16. A kit, comprising:

reagents for performing an assay method configured to detect one or more biomarkers comprising Dashurin, wherein the assay method generates a detectable signal indicative of the presence or amount of a physiologically relevant concentration of Dashurin of SEQ ID NO: 1 and/or immunoreactive fragments thereof.

**Patentansprüche**

1. Verfahren zum Evaluieren des Nierenstatus bei einem Subjekt, umfassend:

Durchführen eines Untersuchungsverfahrens, das dafür konfiguriert ist, einen oder mehrere Biomarker, umfassend Dashurin, in einer Körperflüssigkeitsprobe, die von dem Subjekt erhalten wurde, nachzuweisen, um ein Untersuchungergebnis bereitzustellen, wobei die Untersuchung ein nachweisbares Signal erzeugt, das indikativ für das Vorhandensein oder die Höhe einer physiologisch relevanten Konzentration von Dashurin der SEQ ID NO: 1 und/oder immunreaktiven Fragmenten davon ist; und Korrelieren des Untersuchungsergebnisses mit dem Nierenstatus des Subjekts.

2. Verfahren nach Anspruch 1, wobei der Korrelationsschritt umfasst:

(i) Korrelieren des Untersuchungsergebnisses mit einem oder mehreren aus Risikostratifizierung, Diagnose, Einstufen, Klassifizieren und Kontrollieren des Nierenstatus des Subjekts, oder
(ii) Zuordnen einer Wahrscheinlichkeit von einer oder mehreren zukünftigen Veränderungen des Nierenstatus auf Grundlage des Untersuchungsergebnisses zu dem Subjekt.

3. Verfahren nach Anspruch 2, wobei die eine oder mehreren zukünftige(n) Veränderung(en) des Nierenstatus eines oder mehrere umfassen aus einer zukünftigen Verletzung der Nierenfunktion, einer zukünftigen reduzierten Nierenfunktion, einer zukünftigen Verbesserung der Nierenfunktion und einem zukünftigen akuten Nierenversagen (ARF).

**4.** Verfahren nach Anspruch 2 oder 3, wobei die Wahrscheinlichkeit von einer oder mehreren zukünftigen Veränderungen des Nierenstatus die ist, dass ein Ereignis von Interesse innerhalb eines Zeitraumes von 72 Stunden ab dem Zeitpunkt, an dem die Körperflüssigkeitsprobe von dem Subjekt erhalten wurde, mehr oder weniger wahrscheinlich auftritt.

**5.** Verfahren nach Anspruch 1, wobei das Subjekt kein akutes Nierenversagen hat.

**6.** Verfahren nach Anspruch 1, wobei das Subjekt keinen 1,5-fachen oder höheren Anstieg von Serumkreatinin gegenüber einem Basiswert, der vor dem Zeitpunkt bestimmt wurde, an dem die Körperflüssigkeitsprobe erhalten wurde, erfahren hat, oder wobei das Subjekt eine Urinproduktion von mindestens 0,5 ml/kg/Std. über die 12 Stunden, die dem Zeitpunkt vorausgehen, an dem die Körperflüssigkeitsprobe erhalten wurde, hat.

**7.** Verfahren nach Anspruch 1, wobei das Subjekt keinen Anstieg von Serumkreatinin von 0,3 mg/dl oder höher gegenüber einem Basiswert, der vor dem Zeitpunkt bestimmt wurde, an dem die Körperflüssigkeitsprobe erhalten wurde, erfahren hat, oder wobei das Subjekt (i) keinen 1,5-fachen oder höheren Anstieg von Serumkreatinin gegenüber einem Basiswert, der vor dem Zeitpunkt bestimmt wurde, an dem die Körperflüssigkeitsprobe erhalten wurde, erfahren hat, (ii) eine Urinproduktion von mindestens 0,5 ml/kg/Std. über die 12 Stunden, die dem Zeitpunkt vorausgehen, an dem die Körperflüssigkeitsprobe erhalten wurde, hat, und (iii) keinen Anstieg von Serumkreatinin von 0,3 mg/dl oder mehr gegenüber einem Basiswert, der vor dem Zeitpunkt bestimmt wurde, an dem die Körperflüssigkeitsprobe erhalten wurde, erfahren hat.

**8.** Verfahren nach Anspruch 1, wobei das Subjekt in RIFLE-Stadium 0 oder R ist.

**9.** Verfahren nach Anspruch 8, wobei

(i) das Subjekt in RIFLE-Stadium 0 ist, und der Korrelationsschritt das Zuordnen einer Wahrscheinlichkeit, dass das Subjekt RIFLE-Stadium R oder I innerhalb von 72 Stunden erreichen wird, umfasst, oder
(ii) das Subjekt in RIFLE-Stadium 0 oder R ist, und der Korrelationsschritt das Zuordnen einer Wahrscheinlichkeit, dass das Subjekt RIFLE-Stadium I oder F innerhalb von 72 Stunden oder innerhalb von 48 Stunden oder innerhalb von 24 Stunden erreichen wird, umfasst.

**10.** Verfahren nach Anspruch 1, wobei das Subjekt in RIFLE-Stadium I ist und der Korrelationsschritt das Zuordnen einer Wahrscheinlichkeit, dass das Subjekt RIFLE-Stadium F innerhalb von 72 Stunden oder innerhalb von 48 Stunden oder innerhalb von 24 Stunden erreichen wird, umfasst.

**11.** Verfahren nach Anspruch 1, wobei das/die Untersuchungsergebnis(se) eine gemessene Urin- oder Plasmakonzentration von Dashurin umfasst/umfassen und der Korrelationsschritt umfasst Vergleichen der gemessenen Konzentration mit einer Schwellenwertkonzentration, und
Zuordnen einer erhöhten Wahrscheinlichkeit des Fortschreitens zu einem schlechteren RIFLE-Stadium zu dem Subjekt, in Bezug auf das derzeitige RIFLE-Stadium des Subjekts, wenn die gemessene Konzentration über dem Schwellenwert liegt, oder Zuordnen einer verringerten Wahrscheinlichkeit des Fortschreitens zu einem schlechteren RIFLE-Stadium zu dem Subjekt, in Bezug auf das derzeitige RIFLE-Stadium des Subjekts, wenn die gemessene Konzentration unter dem Schwellenwert liegt, oder
wobei das/die Untersuchungsergebnis(se) eine gemessene Urin- oder Plasmakonzentration von Dashurin umfasst/umfassen und der Korrelationsschritt umfasst Vergleichen der gemessenen Konzentration mit einer Schwellenwertkonzentration, und
Zuordnen einer erhöhten Wahrscheinlichkeit des Fortschreitens zur Notwendigkeit einer Nierenersatztherapie zu dem Subjekt, wenn die gemessene Konzentration über dem Schwellenwert liegt oder Zuordnen einer verringerten Wahrscheinlichkeit des Fortschreitens zur Notwendigkeit einer Nierenersatztherapie, wenn die gemessene Konzentration unter dem Schwellenwert liegt oder
wobei das/die Untersuchungsergebnis(se) eine gemessene Urin- oder Plasmakonzentration von Dashurin umfasst/umfassen und der Korrelationsschritt umfasst Vergleichen der gemessenen Konzentration mit einer Schwellenwertkonzentration, und
Zuordnen einer erhöhten Wahrscheinlichkeit des Fortschreitens zu einem schlechteren RIFLE-Stadium zu dem Subjekt, in Bezug auf das derzeitige RIFLE-Stadium des Subjekts, wenn die gemessene Konzentration über dem Schwellenwert liegt, oder Zuordnen einer verringerten Wahrscheinlichkeit des Fortschreitens zu einem schlechteren RIFLE-Stadium zu dem Subjekt, in Bezug auf das derzeitige RIFLE-Stadium des Subjekts, wenn die gemessene Konzentration unter dem Schwellenwert liegt.

**12.** Verfahren nach Anspruch 5, wobei das/die Untersuchungsergebnis(se) eine gemessene Urin- oder Plasmakonzentration von Dashurin umfasst/umfassen und der Korrelationsschritt umfasst Vergleichen der gemessenen Konzentration mit einer Schwellenwertkonzentration, und

Zuordnen einer erhöhten Wahrscheinlichkeit des Fortschreitens zu akutem Nierenversagen, wenn die gemessene Konzentration über dem Schwellenwert liegt, oder Zuordnen einer verringerten Wahrscheinlichkeit des Fortschreitens zu akutem Nierenversagen zu dem Subjekt, wenn die gemessene Konzentration unter dem Schwellenwert liegt.

**13.** Verfahren nach Anspruch 9, wobei das/die Untersuchungsergebnis(se) eine gemessene Urin- oder Plasmakonzentration von Dashurin umfasst/umfassen und der Korrelationsschritt umfasst Vergleichen der gemessenen Konzentration mit einer Schwellenwertkonzentration, und

Zuordnen einer erhöhten Wahrscheinlichkeit des Fortschreitens zu RIFLE-Stadium R, I oder F zu dem Subjekt, wenn die gemessene Konzentration über dem Schwellenwert liegt, oder Zuordnen einer verringerten Wahrscheinlichkeit des Fortschreitens zu RIFLE-Stadium R, I oder F zu dem Subjekt, wenn die gemessene Konzentration unter dem Schwellenwert liegt.

**14.** Verfahren nach Anspruch 1, wobei das Subjekt zur Beurteilung des Nierenstatus ausgewählt wird anhand

(i) des vorherigen Bestehens von einem oder mehreren bekannten Risikofaktoren für prärenales, immanent renales oder postrenales ARF bei dem Subjekt, oder

(ii) einer vorliegenden Diagnose von einem oder mehreren aus kongestiver Herzinsuffizienz, Präeklampsie, Eklampsie, Diabetes mellitus, Hypertonie, koronarer Arterienkrankheit, Proteinurie, Niereninsuffizienz, glomerulärer Filtration unterhalb des Normbereichs, Zirrhose, Serumkreatinin oberhalb des Normbereichs, Sepsis, Verletzung der Nierenfunktion, verringerter Nierenfunktion oder ARF, oder anhand einer laufenden oder vorangegangenen gößeren Gefäßoperation, Koronararterien-Bypass-Operation oder einer anderen Herzoperation, oder anhand der Exposition gegenüber NSAIDs, Cyclosporinen, Tacrolimus, Aminoglykosiden, Foscarnet, Ethylenglykol, Hämoglobin, Myoglobin, Ifosfamid, Schwermetallen, Methotrexat, Röntgenkontrastmitteln oder Streptozotocin.

**15.** Verwendung von Dashurin der SEQ ID NO: 1 und/oder immunreaktiven Fragmenten davon zur Diagnose, Risikostratifizierung, Prognose, zum Klassifizieren und Kontrollieren des Nierenstatus eines Subjekts, das keine Nierenersatztherapie erhält,

zur Diagnose, Risikostratifizierung, Prognose, zum Klassifizieren und Kontrollieren des Nierenstatus eines Subjekts, bei dem kein akutes Nierenversagen vorliegt, oder

zum Zuordnen einer erhöhten Wahrscheinlichkeit des Fortschreitens zu einem schlechteren RIFLE-Stadium zu dem Subjekt, in Bezug auf das derzeitige RIFLE-Stadium des Subjekts.

**16.** Kit, umfassend:

Reagenzien zum Durchführen eines Untersuchungsverfahrens, das dafür konfiguriert ist, einen oder mehrere Biomarker, umfassend Dashurin, nachzuweisen, wobei das Untersuchungsverfahren ein nachweisbares Signal erzeugt, das indikativ für das Vorhandensein oder die Höhe einer physiologisch relevanten Konzentration von Dashurin der SEQ ID NO: 1 und/oder immunreaktiven Fragmenten davon ist.

**Revendications**

**1.** Un procédé pour évaluer l'état rénal chez un sujet, consistant à :

réaliser un procédé de dosage configuré pour détecter un ou plusieurs biomarqueurs comprenant du Dashurin sur un échantillon de fluide corporel obtenu chez le sujet pour fournir un résultat de dosage, dans lequel le dosage génère un signal détectable indicateur de la présence ou du niveau d'une concentration physiologiquement significative de Dashurin de SEQ ID No : 1 et/ou de fragments immunoréactifs de celui-ci ; et corréler le résultat de dosage avec l'état rénal du sujet.

**2.** Un procédé selon la revendication 1, dans lequel ladite étape de corrélation consiste à :

(i) corréler le résultat de dosage avec un ou plusieurs de stratification de risque, diagnostic, évaluation, classification et surveillance de l'état rénal du sujet, ou

(ii) attribuer une probabilité d'un ou plusieurs changements futurs dans l'état rénal, au sujet sur la base du résultat de dosage.

3. Un procédé selon la revendication 2, dans lequel ledit ou lesdits futurs changements dans l'état rénal comprennent un ou plusieurs d'une lésion de la fonction rénale, une future fonction rénale réduite, une future amélioration de la fonction rénale et une future insuffisance rénale aiguë (IRA).

4. Un procédé selon la revendication 2 ou 3, dans lequel la probabilité d'un ou de plusieurs changements futurs dans l'état rénal est qu'un événement d'intérêt est plus ou moins susceptible de se produire dans une période de 72 heures à partir de l'instant auquel l'échantillon de fluide corporel est obtenu à partir du sujet.

5. Un procédé selon la revendication 1, dans lequel le sujet n'est pas en insuffisance rénale aiguë.

6. Un procédé selon la revendication 1, dans lequel le sujet n'a pas subi une augmentation de 1,5 fois ou supérieure de la créatinine sérique au-dessus d'une valeur de ligne de base déterminée avant le moment auquel l'échantillon de fluide corporel est obtenu, ou dans lequel le sujet a un débit urinaire d'au moins 0,5 ml/kg/hr dans les 12 heures précédent le moment auquel l'échantillon de fluide corporel est obtenu.

7. Un procédé selon la revendication 1, dans lequel le sujet n'a pas subi une augmentation de 0,3 mg/dL ou supérieure de la créatinine sérique au-dessus d'une valeur de ligne de base déterminée avant le moment auquel l'échantillon de fluide corporel est obtenu, ou dans lequel le sujet (i) n'a pas subi une augmentation de 1,5 fois ou supérieure de la créatinine sérique au-dessus d'une valeur de ligne de base déterminée avant le moment auquel l'échantillon de fluide corporel est obtenu, (ii) a un débit urinaire d'au moins 0,5 ml/kg/hr dans les 12 heures précédent le moment auquel l'échantillon de fluide corporel est obtenu, et (iii) n'a pas subi une augmentation de 0,3 mg/dL ou supérieure de la créatinine sérique au-dessus d'une valeur de ligne de base déterminée avant le moment auquel l'échantillon de fluide corporel est obtenu.

8. Un procédé selon la revendication 1, dans lequel le sujet est au stade RIFLE 0 ou R.

9. Un procédé selon la revendication 8, dans lequel

(i) le sujet est au stade RIFLE 0, et ladite étape de corrélation consiste à attribuer une probabilité que le sujet atteindra le stade RIFLE R ou I dans les 72 heures, ou
(ii) dans lequel le sujet est au stade RIFLE 0 ou R, et ladite étape de corrélation consiste à attribuer une probabilité que le sujet atteindra le stade RIFLE I ou F dans les 72 heures ou dans les 48 heures ou dans les 24 heures.

10. Un procédé selon la revendication 1, dans lequel le sujet est au stade RIFLE I, et ladite étape de corrélation consiste à attribuer une probabilité que le sujet atteindra le stade RIFLE F dans les 72 heures ou dans les 48 heures ou dans les 24 heures.

11. Un procédé selon la revendication 1, dans lequel ledit ou lesdits résultats de dosages comprennent une concentration de Dashurin mesurée dans l'urine ou le plasma, et ladite étape de corrélation consiste à comparer ladite concentration mesurée à un seuil de concentration, et attribuer une probabilité accrue de progresser vers un stade RIFLE s'aggravant chez le sujet, liée au stade RIFLE courant du sujet, lorsque la concentration mesurée est au-dessus du seuil, ou attribuer une probabilité diminuée de progresser vers un stade RIFLE s'aggravant chez le sujet, liée au stade RIFLE courant du sujet, lorsque la concentration mesurée est en dessous du seuil, ou
dans lequel ledit ou lesdits résultats de dosages comprennent une concentration de Dashurin mesurée dans l'urine ou le plasma, et ladite étape de corrélation consiste à comparer ladite concentration mesurée à un seuil de concentration, et
attribuer une probabilité accrue de progresser vers un besoin de thérapie de remplacement rénal chez le sujet, lorsque la concentration mesurée est au-dessus du seuil, ou attribuer une probabilité diminuée de progresser vers un besoin de thérapie de remplacement rénal lorsque la concentration mesurée est en dessous du seuil, ou
dans lequel ledit ou lesdits résultats de dosages comprennent une concentration de Dashurin mesurée dans l'urine ou le plasma, et ladite étape de corrélation consiste à comparer ladite concentration mesurée à un seuil de concentration, et
attribuer une probabilité accrue de progresser vers un stade RIFLE s'aggravant chez le sujet, liée au stade RIFLE courant du sujet, lorsque la concentration mesurée est au-dessus du seuil, ou attribuer une probabilité diminuée de progresser vers un stade RIFLE s'aggravant chez le sujet, liée au stade RIFLE courant du sujet, lorsque la concentration mesurée est en dessous du seuil.

12. Un procédé selon la revendication 5, dans lequel ledit ou lesdits résultats de dosages comprennent une concentration de Dashurin mesurée dans l'urine ou le plasma, et ladite étape de corrélation consiste à comparer ladite concentration mesurée à un seuil de concentration, et attribuer une probabilité accrue de progresser vers une insuffisance rénale aiguë lorsque la concentration mesurée est au-dessus du seuil, ou attribuer une probabilité diminuée de progresser vers une insuffisance rénale aiguë chez le sujet lorsque la concentration mesurée est en dessous du seuil.

13. Un procédé selon la revendication 9, dans lequel ledit ou lesdits résultats de dosages comprennent une concentration de Dashurin mesurée dans l'urine ou le plasma, et ladite étape de corrélation consiste à comparer ladite concentration mesurée à un seuil de concentration, et attribuer une probabilité accrue de progresser vers le stade RIFLE R, I ou F chez le sujet, lorsque la concentration mesurée est au-dessus du seuil, ou attribuer une probabilité diminuée de progresser vers le stade RIFLE R, I ou F chez le sujet lorsque la concentration mesurée est en dessous du seuil.

14. Un procédé selon la revendication 1, dans lequel le sujet est choisi pour l'évaluation de l'état rénal en fonction de

(i) la pré-existence chez le sujet d'un ou plusieurs facteurs de risque connus pour une insuffisance rénale aiguë pré-rénale, intrinsèquement rénale ou post-rénale, ou

(ii) un diagnostic existant d'un ou plusieurs parmi l'insuffisance cardiaque congestive, la prééclampsie, l'éclampsie, le diabète sucré, l'hypertension, une maladie coronarienne, la protéinurie, une insuffisance rénale, une filtration glomérulaire en dessous de la plage normale, la cirrhose, la créatinine sérique supérieure à la plage normale, la sepsie, une lésion de la fonction rénale, une fonction rénale amoindrie ou une insuffisance rénale aiguë, ou sur la base du fait de subir ou d'avoir subi une chirurgie vasculaire majeure, un pontage coronarien ou une autre chirurgie cardiaque, ou sur la base d'une exposition à des NSAID, des cyclosporines, du tacrolimus, des aminoglycosides, du foscarnet, de l'éthylène glycol, de l'hémoglobine, de la myoglobine, de l'ifosfamide, des métaux lourds, du méthotrexate, des agents de contraste radio-opaques ou de la streptozotocine.

15. L'utilisation du Dashurin de SEQ ID No°: 1 et/ou des fragments immunoréactifs de celui-ci pour le diagnostic, la stratification de risque, le pronostic, la classification et la surveillance de l'état rénal d'un sujet qui ne reçoit pas une thérapie de remplacement rénal, pour le diagnostic, la stratification de risque, le pronostic, la classification et la surveillance de l'état rénal d'un sujet qui n'est pas en insuffisance rénale aiguë, ou
pour attribuer une probabilité accrue de progresser vers un stade RIFLE s'aggravant chez un sujet, liée au stade RIFLE courant du sujet.

16. Une trousse, comprenant :

des réactifs pour réaliser un procédé de dosage configuré pour détecter un ou plusieurs biomarqueurs comprenant du Dashurin, dans lequel le procédé de dosage génère un signal détectable indicateur de la présence ou du niveau d'une concentration physiologiquement significative de Dashurin de SEQ ID No : 1 et/ou de fragments immunoréactifs de celui-ci.

FIGURE 1

FIGURE 2

liver

ovarial carcinoma

pituitary adenoma

insulinoma

pituitary adenoma

goitre

anaplastic
thyroid carcinoma

C20orf116

β-actin

FIGURE 3

FIGURE 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61232318 A
- US 6664385 B1
- US 2008014644 A1
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

### Non-patent literature cited in the description

- Harrison's Principles of Internal Medicine. McGraw Hill, 1741-1830
- Current Medical Diagnosis & Treatment. McGraw Hill, 2008, 785-815
- Merck Manual
- **PRAUGHT ; SHLIPAK.** *Curr Opin Nephrol Hypertens,* 2005, vol. 14, 265-270
- **CHERTOW et al.** *J Am Soc Nephrol,* 2005, vol. 16, 3365-3370
- **LASSNIGG.** *J Am Soc Nephrol,* 2004, vol. 15, 1597-1605
- **BELLOMO et al.** *Crit Care.,* 2004, vol. 8 (4), R204-12
- **MEHTA et al.** *Crit. Care,* 2007, vol. 11, R31
- **MCCOLLOUGH et al.** *Rev Cardiovasc Med.,* 2006, vol. 7 (4), 177-197
- **HOSTE et al.** RIFLE criteria for acute kidney injury are associated with hospital mortality in critically ill patients: a cohort analysis. *Critical Care,* 2006, vol. 10, R73
- The Immunoassay Handbook. Stockton Press, 1994
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **NELSON ; GRISWOLD.** *Comput. Methods Programs Biomed.,* 1988, vol. 27, 65-8
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **FISCHER et al.** *Intensive Care Med.,* 2003, vol. 29, 1043-51
- Merck Manual of Diagnosis and Therapy. Merck Research Laboratories, 1999
- **BROOKS et al.** *Biochem. J.,* 2000, vol. 346, 155-161
- **HANLEY, J. A. ; MCNEIL, B.J.** The meaning and use of the area under a receiver operating characteristic (ROC) curve. *Radiology,* 1982, vol. 143, 29-36
- **ASANO, K. ; KINZY, T. G. ; MERRICK, W. C. ; HERSHEY, J. W.** Conservation and diversity of eukaryotic translation initiation factor eIF3. *J Biol Chem,* 1997, vol. 272, 1101-1109
- **ASANO, K. ; MERRICK, W. C. ; HERSHEY, J. W.** The translation initiation factor eIF3-p48 subunit is encoded by int-6, a site of frequent integration by the mouse mammary tumor virus genome. *J Biol Chem,* 1997, vol. 272, 23477-23480
- **ASANO, K. ; VORNLOCHER, H. P. ; RICHTER-COOK, N. J. ; MERRICK, W. C. ; HINNEBUSCH, A. G. ; HERSHEY, J. W.** Structure of cDNAs encoding human eukaryotic initiation factor 3 subunits. Possible roles in RNA binding and macromolecular assembly. *J Biol Chem,* 1997, vol. 272, 27042-27052

- **BLOCK, K. L. ; VORNLOCHER, H. P. ; HERSHEY, J. W.** Characterization of cDNAs encoding the p44 and p35 subunits of human translation initiation factor eIF3. *J Biol Chem,* 1998, vol. 273, 31901-31908
- **CASALS, G. ; FILELLA, X. ; AUGE, J. M. ; BEDINI, J. L.** Impact of ultrasensitive cardiac troponin I dynamic changes in the new universal definition of myocardial infarction. *Am J Clin Pathol,* 2008, vol. 130, 964-968
- **DAIBATA, M. ; TAGUCHI, T. ; TAGUCHI, H.** *A novel t(16;20)(q22;p13) in polycythemia vera. Cancer Genet Cytogenet,* 2002, vol. 137, 29-32
- **EWING, R. M. ; CHU, P. ; ELISMA, F. ; LI, H. ; TAYLOR, P. ; CLIMIE, S. ; MCBROOM-CERAJEWSKI, L. ; ROBINSON, M. D. ; O'CONNOR, L. ; LI, M. et al.** Large-scale mapping of human protein-protein interactions by mass spectrometry. *Mol Syst Biol,* 2007, vol. 3, 89
- **HOFMANN, K. ; BUCHER, P.** The PCI domain: a common theme in three multiprotein complexes. *Trends Biochem Sci,* 1998, vol. 23, 204-205
- **JOHNSON, K. R. ; MERRICK, W. C. ; ZOLL, W. L. ; ZHU, Y.** Identification of cDNA clones for the large subunit of eukaryotic translation initiation factor 3. Comparison of homologues from human, Nicotiana tabacum, Caenorhabditis elegans, and Saccharomyces cerevisiae. *J Biol Chem,* 1997, vol. 272, 7106-7113
- **KURIAN, S. M. ; FLECHNER, S. M. ; KAOUK, J. ; MODLIN, C. ; GOLDFARB, D. ; COOK, D. J. ; HEAD, S. ; SALOMON, D. R.** Laparoscopic donor nephrectomy gene expression profiling reveals upregulation of stress and ischemia associated genes compared to control kidneys. *Transplantation,* 2005, vol. 80, 1067-1071
- **LENBURG, M. E. ; LIOU, L. S. ; GERRY, N. P. ; FRAMPTON, G. M. ; COHEN, H. T. ; CHRISTMAN, M. F.** Previously unidentified changes in renal cell carcinoma gene expression identified by parametric analysis of microarray data. *BMC Cancer,* 2003, vol. 3, 31
- **METHOT, N. ; ROM, E. ; OLSEN, H. ; SONENBERG, N.** The human homologue of the yeast Prt1 protein is an integral part of the eukaryotic initiation factor 3 complex and interacts with p170. *J Biol Chem,* 1997, vol. 272, 1110-1116
- **PEI, J. ; BALSARA, B. R. ; LI, W. ; LITWIN, S. ; GABRIELSON, E. ; FEDER, M. ; JEN, J. ; TESTA, J. R.** Genomic imbalances in human lung adenocarcinomas and squamous cell carcinomas. *Genes Chromosomes Cancer,* 2001, vol. 31, 282-287
- **RAPOPORT, T. A.** Protein translocation across the eukaryotic endoplasmic reticulum and bacterial plasma membranes. *Nature,* 2007, vol. 450, 663-669
- **STITZIEL, N. O. ; MAR, B. G. ; LIANG, J. ; WESTBROOK, C. A.** Membrane-associated and secreted genes in breast cancer. *Cancer Res,* 2004, vol. 64, 8682-8687
- **VON HEIJNE, G.** Signal sequences. The limits of variation. *J Mol Biol,* 1985, vol. 184, 99-105
- **XUE, Y. ; YU, F. ; XIN, Y. ; LU, D. ; ZOU, Z. ; GUO, Y. ; XIE, X.** t(8;20)(q22;p13): a novel variant translocation of t(8;21) in acute myeloblastic leukaemia. *Br J Haematol,* 1997, vol. 98, 733-735
- **YAMAMOTO, K. ; HAMAGUCHI, H. ; NAGATA, K. ; KOBAYASHI, M. ; TAKASHIMA, T. ; TANIWAKI, M.** A new complex translocation (15;20;17)(q22;p13;q21) in acute promyelocytic leukemia. *Cancer Genet Cytogenet,* 1998, vol. 101, 89-94